# FASCICULE DE BREVET EUROPEEN

(11) **EP 2 421 961 B1**
(45) Date de publication et mention de la délivrance du brevet: **04.06.2014**
(21) Numéro de dépôt: 10714034.5
(22) Date de dépôt: 22.04.2010
(51) Int. Cl.: C12N 9/10

(54) **4S-IOTA-CARRAGHÉNANE SULFATASE ET SON UTILISATION POUR L'OBTENTION DE L'ALPHA-CARRAGHÉNANE**
4S-IOTA-CARRAGEEN-SULFATASE UND VERWENDUNG DAVON ZUR GEWINNUNG VON ALPHA-CARRAGEEN
4S-IOTA-CARRAGEENAN SULFATASE AND USE THEREOF TO OBTAIN ALPHA-CARRAGEENAN

(30) Priorité: 22.04.2009 FR 0952642
(43) Date de publication de la demande: 29.02.2012
(73) Titulaire: Centre National de la Recherche Scientifique (CNRS), 75016 Paris (FR); Université Pierre et Marie Curie (Paris 6), 75005 Paris (FR)
(72) Inventeur: JONCOUR GENICOT, Sabine, F-29250 SaintPol De Leon (FR); HELBERT, William, F-29680 Roscoff (FR)
(74) Mandataire: Regimbeau
(86) Numéro de dépôt international: PCT/EP2010/055395
(87) Numéro de publication internationale: WO 2010/122127

(56) Documents cités:
- DATABASE UniProt [Online] 8 novembre 2005 (2005-11-08), "SubName: Full=Putative uncharacterized protein;" XP002562258 extrait de EBI accession no. UNIPROT:Q3IKL4 Database accession no. Q3IKL4 cité dans la demande
- ZINOUN M. ET AL.: "EVIDENCE OF SULFOHYDROLASE ACTIVITY IN THE RED ALGA CALLIBLEPHARIS JUBATA" BOTANICA MARINA, DE GRUYTER, NEW YORK, NY, US, vol. 40, 1 janvier 1997 (1997-01-01), pages 49-53, XP000961562 ISSN: 0006-8055
- WONG K. F. ET AL.: "SULFOHYDROLASE ACTIVITY AND CARRAGEENAN BIOSYNTHESIS IN CHONDRUS CRISPUS (RHODOPHYCEAE)" PLANT PHYSIOLOGY, AMERICAN SOCIETY OF PLANT PHYSIOLOGISTS, ROCKVILLE, MD, US, vol. 61, no. 4, 1 janvier 1978 (1978-01-01), pages 663-666, XP000961463 ISSN: 0032-0889
- MICHEL GURVAN ET AL.: "Bioconversion of red seaweed galactans: a focus on bacterial agarases and carrageenases" APPLIED MICROBIOLOGY AND BIOTECHNOLOGY, vol. 71, no. 1, juin 2006 (2006-06), pages 23-33, XP002562259 ISSN: 0175-7598
- GENICOT-JONCOUR S. ET AL.: "The cyclization of the 3,6-anhydro-galactose ring of iota-carrageenan is catalyzed by two D-galactose-2,6-sulfurylases in the red alga Chondrus crispus." PLANT PHYSIOLOGY NOV 2009, vol. 151, no. 3, novembre 2009 (2009-11), pages 1609-1616, XP002598802 ISSN: 1532-2548

### SEQUENCE LISTING

<110> Centre National de Recherche Scientifique (CNRS) Université Pierre et Marie Curie
<120> 4S-iota-carraghénane sulfatase et son utilisation pour l'obtention de l'alpha-carraghénane
<130> D25897
<150> FR0952642
   <151> 2009-04-22
<160> 9
<170> PatentIn version 3.3
<210> 1
   <211> 3114
   <212> DNA
   <213> Pseudoalteromonas haloplanktis
<220>
   <221> misc_feature
   <223> 4S-iota-carraghénane sulfatase
<400> 1
   caacaagacg atgagccaaa atggcaagta gattcaccta aagggcaatt tgttgatgca      60
   tctattagtg tagagcaagg cacatggatg aatgtcgata taagtcctga cggagaaacc     120
   ttagtatttg atttactggg cgatatttac accatgccaa tgagtggtgg taaagcgact     180
   aaaataacct cagacattgc atggcaaatg cagccgcgct ttagcccaga tggcaagcac     240
   attgctttta catcagatca aggcggtggt gacaatattt ggataatgga tgttaacggc     300
   gaaaaccaaa ccgccgtcac tgacgaaaca tttagattac tcaatagccc tgcgtggagc     360
   cctgatggtg attatttagt tgcacgtaag cattttaccg ccagtcgctc gctaggtgcg     420
   ggtgaagttt ggctttatca caaagccggc ggtaaagggg tgcaactgac taaacgtgaa     480
   aacgatcaaa aagacttagg tgaacccatg ttttcaccgg atggtcggta tgtttatttt     540
   tctcacgatg caaccccagg taaaactttt cattattcaa aagactctgt tgccggtatt     600
   tataaaatta agcgttatga ccgtgaaaca ggcgacatag aaaccattat aagtggcatg     660
   ggcggggcaa taagaccgac gccgtcgcca gatggtaaaa aactagcgta cataaagcgt     720
   gatgactttc aaaccagcct gtacttatat gatttaacca gtggtgagca tactaagcta     780
   tacgacaagc tagagcgcga tatgcaagaa acgtgggcga ttcatggcgt atatccgact     840
   attgcgtgga ccccagataa cgaagagctg gtattttggg ctggtggcac ccttcataaa     900
   tttaatgtag ataataaatc agtcagcgat attgcattta aagtagacac tacgaaaaaa     960
   attcaaaaag cagtgcgttt tactcaaaat atcgacactg atgagtttga tgtaaaaatg    1020
   ctgcgtaatg tgcaaattag ccctgacggt gaaaccgcac tttttgaagc attgggctat    1080
   atttacatgc gcgacttaga atcaggaaaa ataaagcgct taactaagca aaccgatcat    1140
   tatgagttat tcccgcagta ttctcgtgat ggcaaaaaca ttgtctacac cacgtgggat 1200
   gataatgaac aaggcaccgt gcgtgtcgtc tctgcacgca gtggtcgggg cgacactatt 1260
   acacaagagc cgggaaaata cgtagagcca acatttagtc cagacggcaa aaccgtggtg 1320
   tatcgtaaag ccagcggcgg caacatttta aaccctaagt ggtcactgca tcctggggta 1380
   tacagtgtta gtgctaaagg cggaaaaagt gagctcattt ctaaaagtgg ctatcaacct 1440
   cagtttggca gtgccaacga tcgtgtttat attatgagcc catggcctaa gccgacatta 1500
   agtgtggtag agcttgaaag taaaaaagta cgtaaactgt atgaatcaga gcacgccact 1560
   gaatttagag tctctcctga tggtgagtat ttagcttttg ctgagcgctt taaagtactt 1620
   gttactccat ttgtagagcg cggcaaaacg ataaatattg ggcccaaaga cagtcaattt 1680
   ccaattgagc agctatctgt acgcgccggt gaaaacatta gctggagtgc aaacagcaat 1740
   aaactgtatt ggacactggg gcctgagctt taccatgcca gtttagaggg gatgtttgcg 1800
   attaataaag cggatgataa agacgttaaa gtaaaaagcg ggactaacat cggctttagt 1860
   aaaaaaatgg ctgaaccgca gggcatgatt gcattaactg gcgctaaaat tattaccatg 1920
   gagggtgaaa aggtcattga aaatggcgtt attgttaccg atggcaaaca tatcaaagcc 1980
   attggcactg ctgctgatgt gagcattcct aagggagcaa aggtcattga tgtaacaggt 2040
   aaaacaatta tgccaggcat tgtagatgcc catgctcatg gctcacaagc cagtgatgaa 2100
   attatcccgc agcaaaattg gaaaaactta gcaggtcttg cgctgggtgt tactactatt 2160
   cacgacccat caaacgatac cactgaaata tttaccgcga gtgaaatgca aaaagcgggg 2220
   atgatcgttg gtccgcgtat tttctctacc ggtactattt tatacggtgc gaatatgcct 2280
   gggtatacct cgcatattga ctcattagat gatgctaaat ttcatttaga gcgtcttaaa 2340
   aaagtgggtg cgtttagtgt taaatcctat aaccaaccgc gcagagagca gcgtcagcaa 2400
   gttatagaag cagggcgaat gcttgaaatg atggtagtcc ctgaaggggg gtcattatta 2460
   caacataact tgagcatggt agtcgatggt catacaggta ttgaacattc aattcctgtt 2520
   gaacatattt atgacgatat taagcagcta tggtcacaaa gtgatgtagg ttacacacct 2580
   acattggtgg ttgcctatgg cggtatttgg ggtgaaaact actggtatga taaaacggac 2640
   gtatggaatc atccacgttt aagtaaattt gtgcctaaaa accaattatt accgcgctca 2700
   atgcgccgag ttaaagcacc cgatcatcac tataatcact ttaacaatgc tcgcgtagcg 2760
   gctgagttac aagatttagg tgtacttgtt aatttgggtg cgcatggcca acgtgagggg 2820
   ttaggtgcgc attgggaaat gtggatgttt gcccaaggcg gcatgacgtc actggaagca 2880
   attagagcat ctacactcga tccggcaaaa tatttaggcc tagataaaaa tgtgggttca 2940
   ttagaggtcg gtaaactggc agatttaatg gttattgatg gcgacccact gaaaaacatt 3000
   cgtgattcag acaaaattga ttacaccatg atcaacggcc gtttgttcga cgcagctacc 3060
   atgaacgaag tgggtaaaaa acagcgtaaa ccactttatt ttgaaaacaa caaa 3114
<210> 2
   <211> 1038
   <212> PRT
   <213> Pseudoalteromonas haloplanktis
<220>
   <221> misc_feature
   <223> 4S-iota-carraghénane sulfatase
<400> 2
   Gln Gln Asp Asp Glu Pro Lys Trp Gln Val Asp Ser Pro Lys Gly Gln
   1 5 10 15
   Phe Val Asp Ala Ser Ile Ser Val Glu Gln Gly Thr Trp Met Asn Val
   20 25 30
   Asp Ile Ser Pro Asp Gly Glu Thr Leu Val Phe Asp Leu Leu Gly Asp
   35 40 45
   Ile Tyr Thr Met Pro Met Ser Gly Gly Lys Ala Thr Lys Ile Thr Ser
   50 55 60
   Asp Ile Ala Trp Gln Met Gln Pro Arg Phe Ser Pro Asp Gly Lys His
   65 70 75 80
   Ile Ala Phe Thr Ser Asp Gln Gly Gly Gly Asp Asn Ile Trp Ile Met
   85 90 95
   Asp Val Asn Gly Glu Asn Gln Thr Ala Val Thr Asp Glu Thr Phe Arg
   100 105 110
   Leu Leu Asn Ser Pro Ala Trp Ser Pro Asp Gly Asp Tyr Leu Val Ala
   115 120 125
   Arg Lys His Phe Thr Ala Ser Arg Ser Leu Gly Ala Gly Glu Val Trp
   130 135 140
   Leu Tyr His Lys Ala Gly Gly Lys Gly Val Gln Leu Thr Lys Arg Glu
   145 150 155 160
   Asn Asp Gln Lys Asp Leu Gly Glu Pro Met Phe Ser Pro Asp Gly Arg
   165 170 175
   Tyr Val Tyr Phe Ser His Asp Ala Thr Pro Gly Lys Thr Phe His Tyr
   180 185 190
   Ser Lys Asp Ser Val Ala Gly Ile Tyr Lys Ile Lys Arg Tyr Asp Arg
   195 200 205
   Glu Thr Gly Asp Ile Glu Thr Ile Ile Ser Gly Met Gly Gly Ala Ile
       210                 215                 220
   Arg Pro Thr Pro Ser Pro Asp Gly Lys Lys Leu Ala Tyr Ile Lys Arg
   225                 230                 235                 240
   Asp Asp Phe Gln Thr Ser Leu Tyr Leu Tyr Asp Leu Thr Ser Gly Glu
                   245                 250                 255
   His Thr Lys Leu Tyr Asp Lys Leu Glu Arg Asp Met Gln Glu Thr Trp
               260                 265                 270
   Ala Ile His Gly Val Tyr Pro Thr Ile Ala Trp Thr Pro Asp Asn Glu
           275                 280                 285
   Glu Leu Val Phe Trp Ala Gly Gly Thr Leu His Lys Phe Asn Val Asp
       290                 295                 300
   Asn Lys Ser Val Ser Asp Ile Ala Phe Lys Val Asp Thr Thr Lys Lys
   305                 310                 315                 320
   Ile Gln Lys Ala Val Arg Phe Thr Gln Asn Ile Asp Thr Asp Glu Phe
                   325                 330                 335
   Asp Val Lys Met Leu Arg Asn Val Gln Ile Ser Pro Asp Gly Glu Thr
               340                 345                 350
   Ala Leu Phe Glu Ala Leu Gly Tyr Ile Tyr Met Arg Asp Leu Glu Ser
           355                 360                 365
   Gly Lys Ile Lys Arg Leu Thr Lys Gln Thr Asp His Tyr Glu Leu Phe
       370                 375                 380
   Pro Gln Tyr Ser Arg Asp Gly Lys Asn Ile Val Tyr Thr Thr Trp Asp
   385                 390                 395                 400
   Asp Asn Glu Gln Gly Thr Val Arg Val Val Ser Ala Arg Ser Gly Arg
                   405                 410                 415
   Gly Asp Thr Ile Thr Gln Glu Pro Gly Lys Tyr Val Glu Pro Thr Phe
               420                 425                 430
   Ser Pro Asp Gly Lys Thr Val Val Tyr Arg Lys Ala Ser Gly Gly Asn
           435                 440                 445
   Ile Leu Asn Pro Lys Trp Ser Leu His Pro Gly Val Tyr Ser Val Ser
       450                 455                 460
   Ala Lys Gly Gly Lys Ser Glu Leu Ile Ser Lys Ser Gly Tyr Gln Pro
   465 470 475 480
   Gln Phe Gly Ser Ala Asn Asp Arg Val Tyr Ile Met Ser Pro Trp Pro
   485 490 495
   Lys Pro Thr Leu Ser Val Val Glu Leu Glu Ser Lys Lys Val Arg Lys
   500 505 510
   Leu Tyr Glu Ser Glu His Ala Thr Glu Phe Arg Val Ser Pro Asp Gly
   515 520 525
   Glu Tyr Leu Ala Phe Ala Glu Arg Phe Lys Val Leu Val Thr Pro Phe
   530 535 540
   Val Glu Arg Gly Lys Thr Ile Asn Ile Gly Pro Lys Asp Ser Gln Phe
   545 550 555 560
   Pro Ile Glu Gln Leu Ser Val Arg Ala Gly Glu Asn Ile Ser Trp Ser
   565 570 575
   Ala Asn Ser Asn Lys Leu Tyr Trp Thr Leu Gly Pro Glu Leu Tyr His
   580 585 590
   Ala Ser Leu Glu Gly Met Phe Ala Ile Asn Lys Ala Asp Asp Lys Asp
   595 600 605
   Val Lys Val Lys Ser Gly Thr Asn Ile Gly Phe Ser Lys Lys Met Ala
   610 615 620
   Glu Pro Gln Gly Met Ile Ala Leu Thr Gly Ala Lys Ile Ile Thr Met
   625 630 635 640
   Glu Gly Glu Lys Val Ile Glu Asn Gly Val Ile Val Thr Asp Gly Lys
   645 650 655
   His Ile Lys Ala Ile Gly Thr Ala Ala Asp Val Ser Ile Pro Lys Gly
   660 665 670
   Ala Lys Val Ile Asp Val Thr Gly Lys Thr Ile Met Pro Gly Ile Val
   675 680 685
   Asp Ala His Ala His Gly Ser Gln Ala Ser Asp Glu Ile Ile Pro Gln
   690 695 700
   Gln Asn Trp Lys Asn Leu Ala Gly Leu Ala Leu Gly Val Thr Thr Ile
   705 710 715 720
   His Asp Pro Ser Asn Asp Thr Thr Glu Ile Phe Thr Ala Ser Glu Met
   725 730 735
   Gln Lys Ala Gly Met Ile Val Gly Pro Arg Ile Phe Ser Thr Gly Thr
   740 745 750
   Ile Leu Tyr Gly Ala Asn Met Pro Gly Tyr Thr Ser His Ile Asp Ser
   755 760 765
   Leu Asp Asp Ala Lys Phe His Leu Glu Arg Leu Lys Lys Val Gly Ala
   770 775 780
   Phe Ser Val Lys Ser Tyr Asn Gln Pro Arg Arg Glu Gln Arg Gln Gln
   785 790 795 800
   Val Ile Glu Ala Gly Arg Met Leu Glu Met Met Val Val Pro Glu Gly
   805 810 815
   Gly Ser Leu Leu Gln His Asn Leu Ser Met Val Val Asp Gly His Thr
   820 825 830
   Gly Ile Glu His Ser Ile Pro Val Glu His Ile Tyr Asp Asp Ile Lys
   835 840 845
   Gln Leu Trp Ser Gln Ser Asp Val Gly Tyr Thr Pro Thr Leu Val Val
   850 855 860
   Ala Tyr Gly Gly Ile Trp Gly Glu Asn Tyr Trp Tyr Asp Lys Thr Asp
   865 870 875 880
   Val Trp Asn His Pro Arg Leu Ser Lys Phe Val Pro Lys Asn Gln Leu
   885 890 895
   Leu Pro Arg Ser Met Arg Arg Val Lys Ala Pro Asp His His Tyr Asn
   900 905 910
   His Phe Asn Asn Ala Arg Val Ala Ala Glu Leu Gln Asp Leu Gly Val
   915 920 925
   Leu Val Asn Leu Gly Ala His Gly Gln Arg Glu Gly Leu Gly Ala His
   930 935 940
   Trp Glu Met Trp Met Phe Ala Gln Gly Gly Met Thr Ser Leu Glu Ala
   945 950 955 960
   Ile Arg Ala Ser Thr Leu Asp Pro Ala Lys Tyr Leu Gly Leu Asp Lys
   965 970 975
   Asn Val Gly Ser Leu Glu Val Gly Lys Leu Ala Asp Leu Met Val Ile
   980 985 990
   Asp Gly Asp Pro Leu Lys Asn Ile Arg Asp Ser Asp Lys Ile Asp Tyr
   995 1000 1005
   Thr Met Ile Asn Gly Arg Leu Phe Asp Ala Ala Thr Met Asn Glu
   1010 1015 1020
   Val Gly Lys Lys Gln Arg Lys Pro Leu Tyr Phe Glu Asn Asn Lys
   1025 1030 1035
<210> 3
   <211> 3114
   <212> DNA
   <213> Pseudoalteromonas carrageenovera
<220>
   <221> misc_feature
   <223> 4S-iota-carraghénane sulfatase
<400> 3
   caacaagacg atgagccaaa atggcaagta gattcaccta aaggccagtt tgttgacgct      60
   gcaattagcg ttgaacaagg cacatggatg aatattgatg taagccctga tggaaaaaca     120
   gttgtgtttg acctgctcgg tgatatttat acaatgccaa tgagtggcgg tacagctaca     180
   aaacttacct cagatattgc atggcagatg cagcctcgct ttagtccaaa tggaaaacac     240
   attgctttta cctcagatca ggggggtggc gataatattt ggataatgga tttaaatggc      300
   gaaaaccaac acgcggttac tgatgaaacc tttaggcttt taaacagccc cgcatggagc     360
   cctgatggcg attacctagt tgcacgtaag cactttactg caagccgctc gttgggagct     420
   ggtgaagtgt ggctttatca taaggcgggt ggtaaaggtg ttcagcttac taagcgcgag     480
   aacgatcaaa aagatttagg tgagccaatg ttctccccag atggacgtta tgtttacttc     540
   tcacatgatg caacgccagg taaaactttt cattattcaa aagattcagt agccggtatt     600
   tataaaatta aacgctacga ccgtgaaacg ggcgagatag aaactgttat tgacactatg     660
   gggggagcga taaggcccac accgtcgccc gatggcaaaa agttagcgta cattaagcgc     720
   gatgactttc aaaccagcct ttatttatac gatttaagca gcggcgagca cactaagctt     780
   tacgataaac ttgagcgtga catgcaagaa acgtgggcaa ttcatggtgt ttatccaacc     840
   attgcatgga cgccagataa tgaagagctg gtattttggg ctggtggtac tattcacaag     900
   ctagatgttg cagacaaatc tgttaaaacc atacctttta aagtgcaaac aagtaaaaaa      960
   attcaaaaag cagtgcgatt tacacaaaat ttagacaccg acgagtttga tgtaaaaatg    1020
   ctgcgaaatg tacaggtaag cccagatggc gcaacggcta tttttgaagc acttggttat    1080
   atttataaac gtgatttaga gtcgggtaag attaaacgtt taactaaaca aactgagcat    1140
   tatgagttat tcccgcaata ctctcgtgat ggtaaaaaaa tcgtatacac cacttggaac    1200
   gataacaagc aaggcacggt acgtgttgta tcgtcgcgca gcggacgtgg cgataccatt 1260
   accacagagc ctggcaagta tgtagagcca acatttagcc caaatggtaa aacggttgtg 1320
   taccgaaaag ccacaggtgg cagcatatta aaccctaagt ggtctttaaa cccaggcatt 1380
   tatagcgtaa gcgttaaagg tggtaaaagt gagctgattt ctaaaagcgg gtatcaacca 1440
   cagtttggtg atgaaagtga tcgcgttttt attatgagcc catggccaaa accaacgctt 1500
   agcgtagtag atcttaaaag taaaaaaata cgtaagcttt atgagtctga gcatgcaact 1560
   gaatttagag tttcaccaga tggtcaatat ttagcgtttg ctgagcggtt taaagtgttt 1620
   gtaacacctt ttgtagaaag tggctcaaca attaatataa gccctaagga tagccagttc 1680
   cctatagagc aattgtctgc tcgtgcgggt gaaaacatta gctggaatac taaaagcaat 1740
   acgctttatt ggactttagg cccagagctt taccatgcaa gtttagaggg aatgtttgca 1800
   attaataaag ccgatgatgc tgactttaaa gtaaaaagtg gcgataacat cggttttagt 1860
   aaaaaaatgg ctaagcctaa aggcatgatt gcgttaaaag gcgcaaaaat tattaccatg 1920
   gatggcgaaa aggtcattga aaatggtgtg ataattacag atggcaagca catcaaatca 1980
   attggtacag ctaatgaagt aaccatacca aaagatgcaa aagtaattga tgtaacaggt 2040
   aaaacgatta tgcctggtat tgttgatgca catgcacatg gctctcaagc aagtgatgaa 2100
   ataattccac agcaaaattg gaaaaacttt gcaggtttag cgctgggtgt cactacaatt 2160
   catgacccat caaatgatac aacagaaata tttactgcca gtgaaatgca aaaggcaggg 2220
   atgattgttg gcccgcgtat tttctctaca gggaccattt tatatggcgc aaatatgccg 2280
   ggttatacgt cacacataga ttcgttagag gatgctaaat ttcatttaga gcgacttaaa 2340
   aaagtggggg cgtttagtgt taaatcgtat aaccaaccac ggcgtgagca gcgccagcaa 2400
   gtaattgaag ccgggcgtga actggaaatg atggtagtgc cagaaggtgg ctcattattg 2460
   caacataact taagtatggt ggttgatggc cacacaggta ttgaacattc tattcctgtt 2520
   gaacatattt acgatgatat aaagcagtta tggtcacaaa gtgatgtagg ctatacacct 2580
   acattagttg ttgcttacgg tggtatatgg ggtgaaaact attggtatga taaaacggat 2640
   gtgtggaacc acccacgttt aagtaaattt gtgcctaaaa atcaattatt accgcgctct 2700
   atgcgccgcg taaaagcgcc tgatcatcac tataatcact ttaataatgc acgtgtggca 2760
   aaagagctgc aagacttggg cgtactggtt aatttaggtg cccatgggca gcgtgaaggg 2820
   cttggagcac attgggaaat gtggatgttc gctcaaggcg gaatgagctc actggaggct 2880
   attagagcat caacgctcga cccagccaaa tatttagggc ttgataaaaa cgtaggctcg 2940
   cttgaagtag gtaaacttgc cgatttaatg gtaatagatg gcaatccact taaaaatatt 3000
   cgagattcgg ataaagttga ttacaccatg atcaatggtc gtttatttga tgcaagtaca 3060
   atgaatgaag tgggtaaaaa acagcgtaaa ccactttatt ttgaaaacaa caaa 3114
<210> 4
   <211> 1038
   <212> PRT
   <213> Pseudoalteromonas carrageenovera
<220>
   <221> misc_feature
   <223> 4S-iota-carraghénane sulfatase
<400> 4
   Gln Gln Asp Asp Glu Pro Lys Trp Gln Val Asp Ser Pro Lys Gly Gln
   1               5                   10                  15
   Phe Val Asp Ala Ala Ile Ser Val Glu Gln Gly Thr Trp Met Asn Ile
               20                  25                  30
   Asp Val Ser Pro Asp Gly Lys Thr Val Val Phe Asp Leu Leu Gly Asp
           35                  40                  45
   Ile Tyr Thr Met Pro Met Ser Gly Gly Thr Ala Thr Lys Leu Thr Ser
       50                  55                  60
   Asp Ile Ala Trp Gln Met Gln Pro Arg Phe Ser Pro Asn Gly Lys His
   65                  70                  75                  80
   Ile Ala Phe Thr Ser Asp Gln Gly Gly Gly Asp Asn Ile Trp Ile Met
                   85                  90                  95
   Asp Leu Asn Gly Glu Asn Gln His Ala Val Thr Asp Glu Thr Phe Arg
               100                 105                 110
   Leu Leu Asn Ser Pro Ala Trp Ser Pro Asp Gly Asp Tyr Leu Val Ala
           115                 120                 125
   Arg Lys His Phe Thr Ala Ser Arg Ser Leu Gly Ala Gly Glu Val Trp
       130                 135                 140
   Leu Tyr His Lys Ala Gly Gly Lys Gly Val Gln Leu Thr Lys Arg Glu
   145                 150                 155                 160
   Asn Asp Gln Lys Asp Leu Gly Glu Pro Met Phe Ser Pro Asp Gly Arg
                   165                 170                 175
   Tyr Val Tyr Phe Ser His Asp Ala Thr Pro Gly Lys Thr Phe His Tyr
               180                 185                 190
   Ser Lys Asp Ser Val Ala Gly Ile Tyr Lys Ile Lys Arg Tyr Asp Arg
           195                 200                 205
   Glu Thr Gly Glu Ile Glu Thr Val Ile Asp Thr Met Gly Gly Ala Ile
       210                 215                 220
   Arg Pro Thr Pro Ser Pro Asp Gly Lys Lys Leu Ala Tyr Ile Lys Arg
   225                 230                 235                 240
   Asp Asp Phe Gln Thr Ser Leu Tyr Leu Tyr Asp Leu Ser Ser Gly Glu
                   245                 250                 255
   His Thr Lys Leu Tyr Asp Lys Leu Glu Arg Asp Met Gln Glu Thr Trp
               260                 265                 270
   Ala Ile His Gly Val Tyr Pro Thr Ile Ala Trp Thr Pro Asp Asn Glu
           275                 280                 285
   Glu Leu Val Phe Trp Ala Gly Gly Thr Ile His Lys Leu Asp Val Ala
       290                 295                 300
   Asp Lys Ser Val Lys Thr Ile Pro Phe Lys Val Gln Thr Ser Lys Lys
   305                 310                 315                 320
   Ile Gln Lys Ala Val Arg Phe Thr Gln Asn Leu Asp Thr Asp Glu Phe
                   325                 330                 335
   Asp Val Lys Met Leu Arg Asn Val Gln Val Ser Pro Asp Gly Ala Thr
               340                 345                 350
   Ala Ile Phe Glu Ala Leu Gly Tyr Ile Tyr Lys Arg Asp Leu Glu Ser
           355                 360                 365
   Gly Lys Ile Lys Arg Leu Thr Lys Gln Thr Glu His Tyr Glu Leu Phe
       370                 375                 380
   Pro Gln Tyr Ser Arg Asp Gly Lys Lys Ile Val Tyr Thr Thr Trp Asn
   385                 390                 395                 400
   Asp Asn Lys Gln Gly Thr Val Arg Val Val Ser Ser Arg Ser Gly Arg
                   405                 410                 415
   Gly Asp Thr Ile Thr Thr Glu Pro Gly Lys Tyr Val Glu Pro Thr Phe
               420                 425                 430
   Ser Pro Asn Gly Lys Thr Val Val Tyr Arg Lys Ala Thr Gly Gly Ser
           435                 440                 445
   Ile Leu Asn Pro Lys Trp Ser Leu Asn Pro Gly Ile Tyr Ser Val Ser
       450                 455                 460
   Val Lys Gly Gly Lys Ser Glu Leu Ile Ser Lys Ser Gly Tyr Gln Pro
   465 470 475 480
   Gln Phe Gly Asp Glu Ser Asp Arg Val Phe Ile Met Ser Pro Trp Pro
   485 490 495
   Lys Pro Thr Leu Ser Val Val Asp Leu Lys Ser Lys Lys Ile Arg Lys
   500 505 510
   Leu Tyr Glu Ser Glu His Ala Thr Glu Phe Arg Val Ser Pro Asp Gly
   515 520 525
   Gln Tyr Leu Ala Phe Ala Glu Arg Phe Lys Val Phe Val Thr Pro Phe
   530 535 540
   Val Glu Ser Gly Ser Thr Ile Asn Ile Ser Pro Lys Asp Ser Gln Phe
   545 550 555 560
   Pro Ile Glu Gln Leu Ser Ala Arg Ala Gly Glu Asn Ile Ser Trp Asn
   565 570 575
   Thr Lys Ser Asn Thr Leu Tyr Trp Thr Leu Gly Pro Glu Leu Tyr His
   580 585 590
   Ala Ser Leu Glu Gly Met Phe Ala Ile Asn Lys Ala Asp Asp Ala Asp
   595 600 605
   Phe Lys Val Lys Ser Gly Asp Asn Ile Gly Phe Ser Lys Lys Met Ala
   610 615 620
   Lys Pro Lys Gly Met Ile Ala Leu Lys Gly Ala Lys Ile Ile Thr Met
   625 630 635 640
   Asp Gly Glu Lys Val Ile Glu Asn Gly Val Ile Ile Thr Asp Gly Lys
   645 650 655
   His Ile Lys Ser Ile Gly Thr Ala Asn Glu Val Thr Ile Pro Lys Asp
   660 665 670
   Ala Lys Val Ile Asp Val Thr Gly Lys Thr Ile Met Pro Gly Ile Val
   675 680 685
   Asp Ala His Ala His Gly Ser Gln Ala Ser Asp Glu Ile Ile Pro Gln
   690 695 700
   Gln Asn Trp Lys Asn Phe Ala Gly Leu Ala Leu Gly Val Thr Thr Ile
   705 710 715 720
   His Asp Pro Ser Asn Asp Thr Thr Glu Ile Phe Thr Ala Ser Glu Met
   725 730 735
   Gln Lys Ala Gly Met Ile Val Gly Pro Arg Ile Phe Ser Thr Gly Thr
   740 745 750
   Ile Leu Tyr Gly Ala Asn Met Pro Gly Tyr Thr Ser His Ile Asp Ser
   755 760 765
   Leu Glu Asp Ala Lys Phe His Leu Glu Arg Leu Lys Lys Val Gly Ala
   770 775 780
   Phe Ser Val Lys Ser Tyr Asn Gln Pro Arg Arg Glu Gln Arg Gln Gln
   785 790 795 800
   Val Ile Glu Ala Gly Arg Glu Leu Glu Met Met Val Val Pro Glu Gly
   805 810 815
   Gly Ser Leu Leu Gln His Asn Leu Ser Met Val Val Asp Gly His Thr
   820 825 830
   Gly Ile Glu His Ser Ile Pro Val Glu His Ile Tyr Asp Asp Ile Lys
   835 840 845
   Gln Leu Trp Ser Gln Ser Asp Val Gly Tyr Thr Pro Thr Leu Val Val
   850 855 860
   Ala Tyr Gly Gly Ile Trp Gly Glu Asn Tyr Trp Tyr Asp Lys Thr Asp
   865 870 875 880
   Val Trp Asn His Pro Arg Leu Ser Lys Phe Val Pro Lys Asn Gln Leu
   885 890 895
   Leu Pro Arg Ser Met Arg Arg Val Lys Ala Pro Asp His His Tyr Asn
   900 905 910
   His Phe Asn Asn Ala Arg Val Ala Lys Glu Leu Gln Asp Leu Gly Val
   915 920 925
   Leu Val Asn Leu Gly Ala His Gly Gln Arg Glu Gly Leu Gly Ala His
   930 935 940
   Trp Glu Met Trp Met Phe Ala Gln Gly Gly Met Ser Ser Leu Glu Ala
   945 950 955 960
   Ile Arg Ala Ser Thr Leu Asp Pro Ala Lys Tyr Leu Gly Leu Asp Lys
   965 970 975
   Asn Val Gly Ser Leu Glu Val Gly Lys Leu Ala Asp Leu Met Val Ile
   980 985 990
   Asp Gly Asn Pro Leu Lys Asn Ile Arg Asp Ser Asp Lys Val Asp Tyr
   995 1000 1005
   Thr Met Ile Asn Gly Arg Leu Phe Asp Ala Ser Thr Met Asn Glu
   1010 1015 1020
   Val Gly Lys Lys Gln Arg Lys Pro Leu Tyr Phe Glu Asn Asn Lys
   1025 1030 1035
<210> 5
   <211> 1060
   <212> PRT
   <213> Pseudoalteromonas haloplanktis
<220>
   <221> misc_feature
   <223> Q3IKL4
<400> 5
   Met Lys Lys Met Leu Gln Thr Val Leu Ala Leu Ser Val Ser Leu Ala
   1 5 10 15
   Leu Gly Asn Ala Gln Ala Gln Gln Asp Asp Glu Pro Lys Trp Gln Val
   20 25 30
   Asp Ser Pro Lys Gly Gln Phe Val Asp Ala Thr Ile Ser Val Asp Gln
   35 40 45
   Gly Thr Trp Met Asn Leu Asp Ile Ser Pro Asp Gly Lys Thr Leu Val
   50 55 60
   Phe Asp Leu Leu Gly Asp Ile Tyr Thr Met Pro Ile Ser Gly Gly Asn
   65 70 75 80
   Ala Thr Gln Leu Thr Ser Asp Ile Ala Trp Gln Met Gln Pro Arg Phe
   85 90 95
   Ser Pro Asn Gly Lys His Ile Ala Phe Thr Ser Asp Gln Gly Gly Gly
   100 105 110
   Asp Asn Ile Trp Ile Met Asp Leu Asn Gly Glu Asn Gln Ser Ala Val
   115 120 125
   Thr Asp Glu Thr Phe Arg Leu Leu Asn Ser Pro Ala Trp Ser Pro Asp
   130 135 140
   Gly Asp Tyr Leu Val Ala Arg Lys His Phe Thr Ala Ser Arg Ser Leu
   145 150 155 160
   Gly Ala Gly Glu Val Trp Leu Tyr His Lys Ala Gly Gly Lys Gly Val
                   165                 170                 175
   Gln Leu Thr Lys Arg Ala Asp Asp Gln Lys Asp Leu Gly Glu Pro Met
               180                 185                 190
   Phe Ser Pro Asp Gly Arg Tyr Val Tyr Phe Ser His Asp Ala Thr Pro
           195                 200                 205
   Gly Lys Thr Phe His Tyr Ser Lys Asp Ser Val Ala Gly Ile Tyr Lys
       210                 215                 220
   Ile Lys Arg Tyr Asp Arg Glu Thr Gly Glu Ile Glu Thr Val Ile Ser
   225                 230                 235                 240
   Gly Met Gly Gly Ala Ile Arg Pro Thr Pro Ser Pro Asp Gly Lys Lys
                   245                 250                 255
   Leu Ala Tyr Ile Lys Arg Asp Asp Phe Gln Thr Ser Leu Tyr Leu Tyr
               260                 265                 270
   Asp Leu Thr Ser Gly Glu His Thr Lys Leu Tyr Asp Lys Leu Glu Arg
           275                 280                 285
   Asp Met Gln Glu Thr Trp Ala Ile His Gly Val Tyr Pro Thr Ile Ala
       290                 295                 300
   Trp Thr Pro Asp Asn Gln Gln Leu Val Phe Trp Ala Gly Gly Thr Ile
   305                 310                 315                 320
   His Lys Leu Asp Val Ser Asp Lys Ser Val Glu Thr Ile Ala Phe Lys
                   325                 330                 335
   Val Gln Thr Thr Lys Lys Ile Gln Lys Ala Val Arg Phe Thr Gln Asn
               340                 345                 350
   Leu Asp Thr Asp Glu Phe Asp Val Lys Met Leu Arg Asn Val Gln Ile
           355                 360                 365
   Ser Pro Asp Gly Glu Thr Ala Ile Phe Glu Ala Leu Gly His Ile Tyr
       370                 375                 380
   Lys Arg Asp Leu Glu Ser Gly Lys Ile Lys Arg Leu Thr Lys Gln Thr
   385                 390                 395                 400
   Asp His Tyr Glu Leu Phe Ala Gln Tyr Ser Arg Asp Gly Lys Lys Ile
                   405                 410                 415
   Val Tyr Thr Thr Trp Asp Asp Asn Glu Gln Gly Gln Val Arg Val Val
   420 425 430
   Ser Ala Ser Ser Gly Arg Gly Asp Thr Ile Thr Glu Gln Pro Gly Lys
   435 440 445
   Tyr Val Glu Pro Thr Phe Ser Pro Asp Gly Lys Thr Val Val Tyr Arg
   450 455 460
   Lys Ala Thr Gly Gly Ser Ile Leu Asn Pro Lys Trp Ser Leu Asn Pro
   465 470 475 480
   Gly Val Tyr Ser Val Ser Thr Lys Gly Gly Lys Ser Glu Leu Ile Ser
   485 490 495
   Lys Asn Gly Tyr Gln Pro Gln Phe Gly Ala Ala Asn Asp Arg Val Tyr
   500 505 510
   Ile Met Ser Pro Trp Pro Lys Pro Thr Leu Ser Val Val Glu Leu Asp
   515 520 525
   Thr Lys Lys Val Arg Lys Leu Tyr Glu Ser Glu His Ala Thr Glu Phe
   530 535 540
   Arg Val Ser Pro Asp Gly Gln Tyr Leu Ala Phe Ala Glu Arg Phe Lys
   545 550 555 560
   Val Phe Val Thr Pro Phe Val Glu Asn Gly Lys Thr Leu Asn Ile Ser
   565 570 575
   Pro Thr Asp Asn Gln Phe Pro Ile Glu Gln Leu Ser Val Arg Ala Gly
   580 585 590
   Glu Asn Ile Ser Trp Ser Ala Asn Ser Asn Lys Leu Tyr Trp Thr Leu
   595 600 605
   Gly Pro Glu Leu Tyr His Ala Ser Leu Glu Gly Met Phe Ala Ile Asn
   610 615 620
   Lys Ala Asp Asp Lys Asp Phe Lys Val Lys Ser Gly Asp Asn Ile Ser
   625 630 635 640
   Phe Ser Lys Lys Met Ala Glu Pro Lys Gly Met Ile Ala Leu Thr Gly
   645 650 655
   Ala Lys Ile Ile Thr Met Asp Gly Glu Lys Val Ile Glu Asn Gly Val
   660 665 670
   Ile Ile Thr Asp Gly Lys His Ile Lys Ala Ile Gly Thr Ala Ala Glu
   675 680 685
   Val Ser Ile Pro Lys Gly Ala Lys Val Val Asp Val Thr Gly Lys Thr
   690 695 700
   Ile Met Pro Gly Ile Val Asp Ala His Ala His Gly Ser Gln Ala Ser
   705 710 715 720
   Asp Glu Ile Ile Pro Gln Gln Asn Trp Lys Asn Phe Ala Gly Leu Ala
   725 730 735
   Leu Gly Val Thr Thr Ile His Asp Pro Ser Asn Asp Thr Ser Glu Ile
   740 745 750
   Phe Thr Ala Ser Glu Met Gln Lys Ala Gly Met Ile Val Gly Pro Arg
   755 760 765
   Ile Phe Ser Thr Gly Thr Ile Leu Tyr Gly Ala Asn Met Pro Gly Tyr
   770 775 780
   Thr Ser His Ile Asp Ser Leu Asp Asp Ala Lys Phe His Leu Glu Arg
   785 790 795 800
   Leu Lys Lys Val Gly Ala Phe Ser Val Lys Ser Tyr Asn Gln Pro Arg
   805 810 815
   Arg Glu Gln Arg Gln Gln Val Ile Glu Ala Gly Arg Glu Leu Gln Met
   820 825 830
   Met Val Val Pro Glu Gly Gly Ser Leu Leu Gln His Asn Leu Ser Met
   835 840 845
   Ile Val Asp Gly His Thr Gly Ile Glu His Ser Ile Pro Val Glu His
   850 855 860
   Ile Tyr Asp Asp Ile Lys Gln Leu Trp Ser Gln Ser Asp Val Gly Tyr
   865 870 875 880
   Thr Pro Thr Leu Val Val Ala Tyr Gly Gly Ile Trp Gly Glu Asn Tyr
   885 890 895
   Trp Tyr Asp Lys Thr Asp Val Trp Asn His Pro Arg Leu Ser Lys Phe
   900 905 910
   Val Pro Lys Asn Gln Leu Leu Pro Arg Ser Met Arg Arg Val Lys Ala
   915 920 925
   Pro Glu His His Tyr Asn His Phe Asn Asn Ala Arg Val Ala Ala Glu
   930 935 940
   Leu Gln Asp Leu Gly Val Leu Val Asn Leu Gly Ala His Gly Gln Arg
   945 950 955 960
   Glu Gly Leu Gly Ala His Trp Glu Met Trp Met Phe Ala Gln Gly Gly
   965 970 975
   Met Thr Pro Leu Glu Ala Ile Arg Ala Ser Thr Leu Asp Pro Ala Lys
   980 985 990
   Tyr Leu Gly Leu Asp Lys Asn Val Gly Ser Leu Glu Val Gly Lys Leu
   995 1000 1005
   Ala Asp Leu Met Val Ile Asp Gly Asp Pro Leu Ser Asn Ile Arg
   1010 1015 1020
   Asp Ser Asp Lys Ile Asp Tyr Thr Met Ile Asn Gly Arg Leu Phe
   1025 1030 1035
   Asn Ala Ala Thr Met Val Glu Val Gly Lys Lys Gln Arg Lys Pro
   1040 1045 1050
   Leu Tyr Phe Glu Asn Asn Lys
   1055 1060
<210> 6
   <211> 23
   <212> PRT
   <213> Pseudoalteromonas tunicata
<220>
   <221> misc_feature
   <223> Peptide signal de la protéine putative non caractérisée A4CBN2
<400> 6
   Met Lys Lys Phe Ile Tyr Ser Ser Val Ala Phe Ala Val Ala Met Thr
   1 5 10 15
   Phe Ser Pro Ala Ser Phe Ala
   20
<210> 7
   <211> 27
   <212> PRT
   <213> Shewanella denitrificans
<220>
   <221> misc_feature
   <223> Peptide signal de l'Amidohydrolase (précurseur) Q12TB8
<400> 7
   Met Leu Met Leu Ser Ile Lys Phe Thr Pro Leu Tyr Thr Ala Ile Ala
   1               5                   10                  15
   Leu Thr Leu Gly Cys Ser Ser Leu Val Tyr Ala
               20                  25
<210> 8
   <211> 22
   <212> PRT
   <213> Alteromonodales bacterium TW-7
<220>
   <221> misc_feature
   <223> Peptide signal de la protéine putative non caractérisée AOY053
<400> 8
   Met Lys Lys Leu Leu His Thr Ala Leu Ala Leu Ser Val Ser Leu Ala
   1               5                   10                  15
   Leu Gly Gln Ala His Ala
               20
<210> 9
   <211> 22
   <212> PRT
   <213> Pseudoalteromonas haloplanktis
<220>
   <221> misc_feature
   <223> Peptide signal de la protéine putative non caractérisée Q3IKL4
<400> 9
   Met Lys Lys Met Leu Gln Thr Val Leu Ala Leu Ser Val Ser Leu Ala
   1               5                   10                  15
   Leu Gly Asn Ala Gln Ala
               20

## Description

La présente invention a pour objet la 4S-iota-carraghénane sulfatase et son utilisation pour la conversion partielle ou totale du iota-carraghénane en iota-/alpha-carraghénane ou en alpha-carraghénane. Elle a également pour objet le procédé d'extraction de cette enzyme à partir d'une population bactérienne *Pseudoalteromonas,* ou bien à partir d'algues rouges marines. Enfin, la présente invention concerne l'utilisation de la 4S-iota-carraghénane sulfatase pour la préparation d'un agent de texture comprenant de l'alpha-carraghénane.

Les carraghénanes définissent des familles de galactanes sulfatés isolés de la matrice extra-cellulaire des algues rouges marines. Ces polysaccharides anioniques ont des propriétés rhéologiques uniques et sont utilisés comme agents de texture en industrie agro-alimentaire, parapharmacie et cosmétique. Les carraghénanes sont composés d'un enchaînement de D-galactoses liés alternativement par des liaisons alpha (1-3) et bêta (1-4). Ces polysaccharides se distinguent par la présence ou non d'un pont 3,6 anhydro sur le résidu galactose lié en alpha (1-3) et par leur taux de sulfatation.

Les carraghénanes possèdent une grande diversité de structures chimiques qui sont corrélées à un large spectre de leurs propriétés fonctionnelles. Par conséquent, à chaque source d'algues correspond des propriétés et des applications qui leurs sont propres. Par ailleurs, en fonction de la structure du carraghénane (par exemple alpha- ou iota-carraghénane) et de l'origine de l'algue (cultivée ou collectée dans le milieu naturel), l'abondance et le coût de ces macromolécules sont très variables. Le tonnage des carraghénanes exploités est limité par la quantité d'algues rouges disponibles. S'agissant de l'alpha-carraghénane, ce polysaccharide a été observé dans l'algue *Catenella nipae.* Dans la nature, l'alpha-carraghénane est toujours associé à du iota-carraghénane. On peut citer l'article de *Zablackis et Santos (1986),* qui décrit pour la première fois la présence d'alpha-carraghénane et le travail de *Falshaw et al. (1996),* qui réalisent l'analyse structurale du polysaccharide.

Dans ce contexte, l'utilisation d'enzymes pour manipuler la structure chimique des carraghénanes permet de contrôler l'approvisionnement et les propriétés fonctionnelles des carraghénanes.

Dans la présente invention, les Inventeurs ont identifié à partir d'extraits de bactéries marines une sulfatase agissant sur le iota-carraghénane, la 4S-iota-carraghénane sulfatase. Cette enzyme permet de catalyser la désulfatation spécifique en position 4 du iota-carraghénane, conduisant en une seule étape à de l'alpha-carraghénane ou à une structure hybride de type alpha-/iota-carraghénane. La réaction s'effectue sans modification apparente du poids moléculaire du carraghénane, permettant ainsi une amélioration notable des propriétés de gélification. La 4S-iota-carraghénane sulfatase a été purifiée et séquencée.

Les Inventeurs ont ainsi pu déterminer que la protéine hypothétique Q3IKL4 (SEQ ID N°5) de la bactérie marine *Pseudoalteromonas haloplanktis,* dont le génome a été entièrement séquencé, et qui est référencée dans la banque protéique TrEMBL accessible sur Internet à l'adresse http://www.ebi.ac.uk/trembl/, correspond en fait à la 4S-iota-carraghénane sulfatase.

La présente invention offre de nombreux avantages pour les raisons suivantes :
- L'invention offre la possibilité de s'affranchir de certaines ressources d'algues dont les coûts deviennent très élevés.
- L'invention permet de produire des carraghénanes de structures hybrides à partir de carraghénanes extraits d'algues cultivées (peu chères).
- L'invention permet également de produire l'alpha-carraghénane, qui est une structure peu abondante, voire inexistante dans la nature.
- Grâce au séquençage de la 4S-iota-carraghénane sulfatase, il est maintenant possible de produire et d'exploiter cette enzyme à grande échelle.

Ainsi, selon un premier aspect, la présente invention a pour objet un polypeptide isolé comprenant une séquence amino-acide ayant au moins 65 % d'identité avec la séquence SEQ ID N°2 ou la séquence SEQ ID N°4.

La présente invention a également pour objet un polypeptide isolé consistant en une séquence amino-acide ayant au moins 65 % d'identité avec la séquence SEQ ID N°2 ou la séquence SEQ ID N°4.

Dans la présente invention, on entend par polypeptide isolé comprenant ou consistant en une séquence amino-acide ayant au moins 65 % d'identité, de préférence au moins 70 %, 75 %, 80 %, 85 %, 90 %, 93 %, 94 %, 95 %, 96 %, 97 %, 98 %, 99 % d'identité avec la séquence SEQ ID N°2 ou la séquence SEQ ID N°4, un polypeptide qui possède la fonction 4S-iota-carraghénane sulfatase. La 4S-iota-carraghénane sulfatase est une enzyme qui permet de convertir le iota-carraghénane en alpha-carraghénane par désulfatation (élimination d'un groupement SO₃₋) spécifique en position 4 du iota-carraghénane (voir schéma ci-dessous). Le poids moléculaire de la 4S-iota-carraghénane sulfatase est de 110 kDaltons environ.

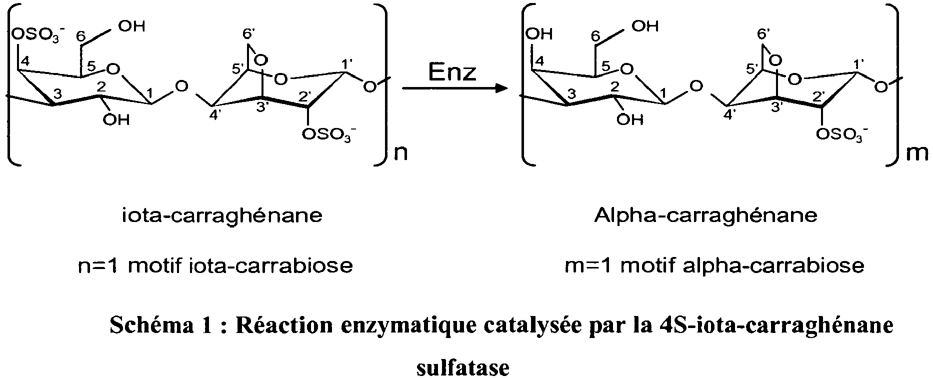

Le iota-carraghénane correspond à au moins un motif iota-carrabiose, de préférence un enchaînement de motifs iota-carrabiose. On parlera d'oligomères ou de polymères de iota-carraghénane en fonction du nombre n d'unités de répétition du motif iota-carrabiose.

Le(s) motif(s) iota-carrabiose peu(ven)t être associé(s) à au moins un autre motif de répétition, formant ainsi un iota-carraghénane hybride, voire un oligo-iota-carraghénane hybride ou un copolymère de iota-carraghénane lorsque plusieurs motifs iota-carrabiose sont associés à d'autres motifs de répétition au sein d'une même chaîne de polysaccharides.

Dans la présente invention, les termes polypeptide, séquence polypeptidique, peptide, séquence peptidique, protéine, séquence protéique, séquence amino-acide sont interchangeables.

Il doit être compris ici que l'invention ne concerne pas la 4S-iota-carraghénane sulfatase sous forme naturelle, c'est-à-dire prise dans son environnement naturel, mais la 4S-iota-carraghénane sulfatase isolée et/ou purifiée à partir de sources naturelles, ou bien obtenue par recombinaison génétique ou synthèse chimique.

Par « pourcentage d'identité » entre deux séquences d'acide nucléique ou d'acide aminé au sens de la présente invention, on entend désigner un pourcentage de nucléotides ou d'acides aminés identiques entre les deux séquences à comparer, obtenu après le meilleur alignement (alignement optimal), ce pourcentage étant purement statistique et les différences entre les deux séquences étant réparties au hasard et sur toute leur longueur. Les comparaisons de séquences entre deux séquences d'acide nucléique ou d'acide aminé sont traditionnellement réalisées en comparant ces séquences après les avoir alignées de manière optimale, ladite comparaison pouvant être réalisée par segment ou par « fenêtre de comparaison ». L'alignement optimal des séquences pour la comparaison peut être réalisé, outre manuellement, au moyen de l'algorithme d'homologie locale de Smith et Waterman (1981) [Ad. App. Math. 2:482], au moyen de l'algorithme d'homologie locale de Neddleman et Wunsch (1970) [J. Mol. Biol. 48:443], au moyen de la méthode de recherche de similarité de Pearson et Lipman (1988) [Proc. Natl. Acad. Sci. USA 85:2444], au moyen de logiciels informatiques utilisant ces algorithmes (GAP, BESTFIT, FASTA et TFASTA dans le Wisconsin Genetics Software Package, Genetics Computer Group, 575 Science Dr., Madison, WI, ou encore par les logiciels de comparaison BLAST N ou BLAST P).

Le pourcentage d'identité entre deux séquences d'acide nucléique ou d'acide aminé est déterminé en comparant ces deux séquences alignées de manière optimale dans laquelle la séquence d'acide nucléique ou d'acide aminé à comparer peut comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions dans la fenêtre de comparaison et en multipliant le résultat obtenu par 100 pour obtenir le pourcentage d'identité entre ces deux séquences.

Par exemple, on pourra utiliser le programme BLAST, « BLAST2 séquences » (Tatusova et al., « Blast 2 sequences - a new tool for comparing protein and nucleotide sequences », FEMS Microbiol., 1999 Lett. 174:247-250) disponible sur le site http://blast.ncbi.nlm.nih.gov/Blast.cgi (lien « Specialized BLAST », « Align two sequences using BLAST (bl2seq) »), le pourcentage d'identité entre les deux séquences à comparer étant calculé directement par le programme.

Par séquence d'acide aminé présentant ou ayant au moins 65 % d'identité, de préférence au moins 70 %, 75 %, 80 %, 85 %, 90 %, 93 %, 94 %, 95 %, 96 %, 97 %, 98 %, 99 % d'identité avec une séquence d'acide aminé de référence, on préfère celles présentant par rapport à la séquence de référence, certaines modifications, en particulier une délétion, addition ou substitution d'au moins un acide aminé, une troncation ou un allongement. Dans le cas d'une substitution d'un ou plusieurs acide(s) aminé(s) consécutif(s) ou non consécutif(s), on préfère les substitutions dans lesquelles les acides aminés substitués sont remplacés par des acides aminés « équivalents ». L'expression « acides aminés équivalents » vise ici à désigner tout acide aminé susceptible d'être substitué à l'un des acides aminés de la structure de base sans cependant modifier essentiellement la fonction 4S-iota-carraghénane sulfatase.

Ces acides aminés peuvent être déterminés soit en s'appuyant sur leur homologie de structure avec les acides aminés auxquels ils se substituent, soit sur des résultats d'essais comparatifs de la fonction 4S-iota-carraghénane sulfatase entre les différentes séquences susceptibles d'être effectués.

A titre d'exemple non limitatif, le tableau 1 ci-dessous reprend les possibilités de substitution susceptibles d'être effectuées sans qu'il en résulte une modification approfondie de la fonction de la 4S-iota-carraghénane sulfatase, les substitutions inverses étant naturellement envisageables dans les mêmes conditions.

**Tableau 1**

| Résidu originel | Substitution(s) |
|---|---|
| Ala (A) | Val, Gly, Pro |
| Arg (R) | Lys, His |
| Asn (N) | Gln |
| Asp (D) | Glu |
| Cys (C) | Ser |
| Gln (Q) | Asn |
| Glu (E) | Asp |
| Gly (G) | Ala |
| His (H) | Arg |
| Ile (I) | Leu |
| Leu (L) | Ile, Val, Met |
| Lys (K) | Arg |
| Met (M) | Leu |
| Phe (F) | Tyr |
| Pro (P) | Ala |
| Ser (S) | Thr, Cys |
| Thr (T) | Ser |
| Trp (W) | Tyr |
| Tyr (Y) | Phe, Trp |
| Val (V) | Leu, Ala |

Le polypeptide isolé selon la présente invention peut comprendre, en plus de la séquence amino-acide ayant au moins 65 % d'identité avec la séquence SEQ ID N°2 ou la séquence SEQ ID N°4, au moins une autre séquence peptidique telle qu'un peptide signal ou un module de liaison à un hydrate de carbone (ou CBM pour « carbohydrate-binding module »). De tels modules sont bien connus de l'homme de l'art et sont notamment accessibles sur Internet à l'adresse www.cazy.org/. Il peut également s'agir d'une protéine telle qu'une enzyme, par exemple une carraghénase, auquel cas on parlera de polypeptide de fusion. D'autres possibilités peuvent être envisagées et la liste des autres séquences peptidiques incluses dans le polypeptide selon l'invention n'est pas limitative. Lorsque le polypeptide isolé selon la présente invention comprend également un peptide signal, ledit peptide signal a pour fonction d'exporter la protéine de séquence amino-acide ayant au moins 65 % d'identité avec la séquence SEQ ID N°2 ou la séquence SEQ ID N°4, vers un compartiment cellulaire particulier, voire à l'extérieur d'une cellule. Dans la présente invention on préférera les peptides signaux des carraghénases, avantageusement les peptides signaux des carraghénases de *Pseudoalteromonas haloplanktis, Pseudoaltenomonas carrageenovora, Pseudoalteromonas tunicata, Alteromonadales bacterium,* ou *Shewanella denitrificans.* De manière encore plus préférée, le peptide signal est choisi dans le groupe constitué par les peptides de séquence SEQ ID N°6, SEQ ID N°7, SEQ ID N°8 et SEQ ID N°9.

**Tableau 2**

| **Organisme** | **Séquence du peptide signal** | **Numéro de séquence** |
|---|---|---|
| *Pseudoalteromonas tunicata* | MKKFIYSSVAFAVAMTFSPASFA | SEQ ID N°6 |
| *Shewanella denitrificans* | MLMLSIKFTPLYTAIALTLGCSSLVY A | SEQ ID N°7 |
| *Alteromonodales bacterium TW-7* | MKKLLHTALALSVSLALGQAHA | SEQ ID N°8 |
| *Pseudoalteromonas haloplanktis* | MKKMLQTVLALSVSLALGNAQA | SEQ ID N°9 |

De préférence, le polypeptide isolé selon la présente invention comprenant ou consistant en la séquence amino-acide ayant au moins 65 % d'identité avec SEQ ID N°2 ou SEQ ID N°4, a une taille comprise entre 1025 et 1070 acides aminés, avantageusement entre 1027 et 1060 acides aminés, encore plus avantageusement entre 1028 et 1055 acides aminés, encore plus avantageusement entre 1030 et 1045 acides aminés, encore plus avantageusement entre 1035 et 1040 acides aminés, de manière préférée entre toutes de 1038 acides aminés.

Selon une autre préférence, la séquence du polypeptide isolé selon la présente invention comprenant ou consistant en la séquence amino-acide ayant au moins 65 % d'identité avec SEQ ID N°2 ou SEQ ID N°4, correspond à celle d'une 4S-iota-carraghénane sulfatase de *Pseudoalteromonas.*

Egalement de manière préférée, la séquence du polypeptide isolé selon la présente invention comprenant ou consistant en la séquence amino-acide ayant au moins 65 % d'identité avec SEQ ID N°2 ou SEQ ID N°4, correspond à celle d'une 4S-iota-carraghénane sulfatase de *Pseudoalteromonans* et a une taille comprise entre 1025 et 1055 acides aminés, avantageusement entre 1030 et 1045 acides aminés, entre 1035 et 1040 acides aminés, de manière préférée entre toutes de 1038 acides aminés.

De manière encore plus préférée, la présente invention a pour objet un polypeptide isolé comprenant une séquence amino-acide ayant au moins 95 % d'identité avec une séquence amino-acide choisie dans le groupe constitué par les séquences SEQ ID N°2 et SEQ ID N°4.

De manière également préférée, la présente invention a pour objet un polypeptide isolé consistant en une séquence amino-acide ayant au moins 95 % d'identité avec une séquence amino-acide choisie dans le groupe constitué par les séquences SEQ ID N°2 et SEQ ID N°4.

De manière préférée entre toutes, la présente invention a également pour objet un polypeptide isolé comprenant une séquence amino-acide choisie dans le groupe constitué par les séquences SEQ ID N°2 et SEQ ID N°4.

Egalement de manière préférée entre toutes, la présente invention a pour objet un polypeptide insolé consistant en une séquence amino-acide choisie dans le groupe constitué par les séquences SEQ ID N°2 et SEQ ID N°4.

Selon un autre aspect, la présente invention a pour objet un polynucléotide isolé choisi parmi les polynucléotides suivants :
a) un polynucléotide, ADN ou ARN, codant le polypeptide selon la présente invention tel que défini ci-dessus ; et
b) un polynucléotide complémentaire d'un polynucléotide tel que défini en a).

Par polynucléotide, séquence de polynucléotide, acide nucléique, séquence nucléique ou d'acide nucléique, termes qui sont désignés indifféremment dans la présente description, on entend désigner un enchaînement précis de nucléotides, modifiés ou non, permettant de définir un fragment ou une région d'un acide nucléique, comportant ou non des nucléotides non naturels, et pouvant correspondre aussi bien à un ADN double brin, un ADN simple brin, que des produits de transcription desdits ADN.

Il doit aussi être compris ici que la présente invention ne concerne pas les séquences nucléotidiques dans leur environnement chromosomique naturel, c'est-à-dire à l'état naturel. Il s'agit de séquences qui ont été isolées et/ou purifiées, c'est-à-dire qu'elles ont été prélevées directement ou indirectement, par exemple par copie, leur environnement ayant été au moins partiellement modifié. On entend ainsi également désigner ici les acides nucléiques isolés obtenus par recombinaison génétique ou par synthèse chimique.

De préférence, le polynucléotide qui code le polypeptide selon la présente invention comprend une séquence nucléotidique ayant au moins 65 % d'identité, de préférence au moins 70 %, 75 %, 80 %, 85 %, 90 %, 93 %, 94 %, 95 %, 96 %, 97 %, 98 %, 99 % d'identité avec la séquence SEQ ID N°1 ou la séquence SEQ ID N°3.

Selon une autre préférence, le polynucléotide qui code le polypeptide selon la présente invention consiste en une séquence nucléotidique ayant au moins 65 % d'identité, de préférence au moins 70 %, 75 %, 80 %, 85 %, 90 %, 93 %, 94 %, 95 %, 96 %, 97 %, 98 %, 99 % d'identité avec la séquence SEQ ID N°1 ou la séquence SEQ ID N°3.

Par polynucléotide présentant au moins 65 % d'identité, de préférence au moins 70 %, 75 %, 80 %, 85 %, 90 %, 93 %, 94 %, 95 %, 96 %, 97 %, 98 %, 99 % d'identité avec une séquence, on entend désigner les polynucléotides présentant, par rapport au polynucléotide de référence, certaines modifications comme en particulier une délétion, une troncation, un allongement, une fusion chimérique et/ou une substitution, notamment ponctuelle. Il s'agit de préférence de séquences codant les mêmes séquences aminoacides que la séquence de référence, ceci lié à la dégénérescence du code génétique, ou de séquences complémentaires qui sont susceptibles de s'hybrider spécifiquement avec les séquences nucléotidiques de référence, de préférence dans des conditions de forte stringence.

Une hybridation dans des conditions de forte stringence signifie que les conditions de température et de force ionique sont choisies de telle manière qu'elles permettent le maintien de l'hybridation entre deux fragments d'ADN complémentaires.

De manière davantage préférée, la séquence nucléotidique est choisie dans le groupe constitué par la séquence SEQ ID N°1 et la séquence SEQ ID N°3.

La présente invention est également relative à un vecteur d'expression comprenant un polynucléotide selon la présente invention tel que défini ci-dessus.

L'invention vise notamment les vecteurs de clonage et/ou d'expression qui contiennent une séquence nucléotidique selon l'invention.

Les vecteurs selon l'invention comportent de préférence des éléments qui permettent l'expression et/ou la sécrétion des séquences nucléotidiques dans une cellule hôte déterminée. Le vecteur doit alors comporter un promoteur, des signaux d'initiation et de terminaison de la traduction, ainsi que des régions appropriées de régulation de la transcription. Il doit pouvoir être maintenu de façon stable dans la cellule hôte et peut éventuellement posséder des signaux particuliers qui spécifient la sécrétion de la protéine traduite. Ces différents éléments sont choisis et optimisés par l'homme du métier en fonction de l'hôte cellulaire utilisé. A cet effet, les séquences nucléotidiques selon l'invention peuvent être insérées dans des vecteurs à réplication autonome au sein de l'hôte choisi, ou être des vecteurs intégratifs de l'hôte choisi.

De tels vecteurs sont préparés par des méthodes couramment utilisées par l'homme du métier, et les clones résultants peuvent être introduits dans un hôte approprié par des méthodes standard, telles que la lipofection, l'électroporation, le choc thermique, ou des méthodes chimiques.

Les vecteurs selon l'invention sont par exemple des vecteurs d'origine plasmidique ou virale. Ils sont utiles pour transformer des cellules hôtes afin de cloner ou d'exprimer les séquences nucléotidiques selon l'invention.

L'invention comprend également une cellule hôte comprenant un vecteur selon la présente invention ou transformée par un vecteur selon la présente invention.

L'hôte cellulaire peut être choisi parmi des systèmes procaryotes ou eucaryotes, par exemple les cellules bactériennes telles que E. *coli* ou *Bacillus,* mais également des cellules de levure ou des cellules animales.

L'invention concerne également les organismes qui contiennent une cellule transformée selon l'invention, tels que des algues rouges, des micro-algues ou des plantes terrestres.

Selon un autre aspect, l'invention a pour objet un procédé de production d'un polypeptide selon la présente invention comprenant les étapes suivantes :
a) la culture dans un milieu et conditions de culture appropriés d'une population de cellules hôtes selon l'invention ; et
b) la récupération desdits polypeptides ainsi produits à partir du milieu de culture ou de la population de cellules hôtes cultivées.

Les cellules transformées selon l'invention sont utilisables dans des procédés de préparation de polypeptides recombinants selon l'invention. Les procédés de préparation d'un polypeptide selon l'invention sous forme recombinante, caractérisés en ce qu'ils mettent en oeuvre un vecteur et/ou une cellule transformée par un vecteur selon l'invention, sont eux-mêmes compris dans la présente invention. De préférence, on cultive une cellule transformée par un vecteur selon l'invention dans des conditions qui permettent l'expression dudit polypeptide et on récupère ledit polypeptide recombinant.

Ainsi qu'il a été dit, l'hôte cellulaire peut être choisi parmi des systèmes procaryotes ou eucaryotes. En particulier, il est possible d'identifier des séquences nucléotidiques selon l'invention, facilitant la sécrétion dans un tel système procaryote ou eucaryote. Un vecteur selon l'invention portant une telle séquence peut donc être utilisé pour la production de protéines recombinantes, destinées à être sécrétées. En effet, la purification de ces protéines recombinantes d'intérêt sera facilitée par le fait qu'elles sont présentes dans le surnageant de la culture cellulaire plutôt qu'à l'intérieur des cellules hôtes. Alternativement, s'agissant des systèmes procaryotes, on peut faire surexprimer les protéines dans l'espace périplasmique des bactéries, ce qui permet d'éviter de manipuler les volumes parfois importants des milieux de culture.

On peut également préparer les polypeptides selon l'invention par synthèse chimique. Un tel procédé de préparation est également un objet de l'invention. L'homme du métier connaît les procédés de synthèse chimique, par exemple les techniques mettant en oeuvre des phases solides (voir notamment Steward et al., 1984, « Solid phase peptide synthesis », Pierce Chem. Company, Rockford, 111, 2éme éd.) ou des techniques utilisant des phases solides partielles, par condensation de fragments ou par une synthèse en solution classique. Les polypeptides obtenus par synthèse chimique et pouvant comporter des acides aminés non naturels correspondants sont également compris dans l'invention.

Les polypeptides susceptibles d'être obtenus par un procédé de production selon la présente invention sont également compris dans l'invention.

Selon un autre aspect, l'invention a pour objet un procédé d'obtention d'une 4S-iota-carraghénane sulfatase à partir d'une population bactérienne susceptible de contenir la 4S-iota-carraghénane sulfatase comprenant :
(a) la mise en culture de la population bactérienne dans un milieu et conditions de culture appropriés pour l'obtention de la 4S-iota-carraghénane sulfatase dans le milieu de culture, et
(b) l'obtention de la 4S-iota-carraghénane sulfatase à partir du milieu de culture obtenu à l'étape (a).

Dans la présente invention, on entend par population bactérienne susceptible de contenir la 4S-iota-carraghénane sulfatase une population bactérienne dont on suppose qu'elle exprime la 4S-iota-carraghénane sulfatase. Il s'agit de préférence d'une population bactérienne ayant une activité carraghénase, c'est-à-dire une activité permettant de dégrader un carraghénane (« population bactérienne carraghénolytique »). De telles bactéries sont notamment décrites dans l'article scientifique *Michel et al, 2006.* La population bactérienne ayant une activité carraghénase est de préférence une population bactérienne appartenant à la classe *Gammaproteobacteria, Flavobacteria* ou *Sphingobacteria.* De préférence, il s'agit d'une population bactérienne appartenant au genre *Alteromonas, Pseudoalteromonas, Cobetia* ou *Zobellia.* De manière encore plus préférée, la population bactérienne appartient au genre *Pseudoalteromonas.*

De préférence, ladite population bactérienne contient de la 4S-iota-carraghénane sulfatase.

De préférence, l'invention a pour objet le procédé d'obtention de la 4S-iota-carraghénane sulfatase à partir de la population bactérienne *Pseudoalteromonas haloplanktis* et/ou *Pseudoalteromonas carrageenovora.*

Un milieu de culture approprié pour une population bactérienne selon l'invention est le milieu Zobell (voir la composition dans la partie Exemples ci-dessous). Il peut également s'agir du milieu de culture DIFCO 2216 (« bacto marine broth »), dont la composition est généralement la suivante : Bacto peptone 5,00 g, extrait de levure 1,00 g, Fe(III) citrate 0,10 g, NaCl 19,45 g, MgC₂ (anhydre) 5,90 g, Na₂SO₄ 3,24 g, CaCl₂ 1,80 g, KCl 0,55 g, Na₂CO₃ 0,16 g, KBr 0,08 g, SrCl₂ 34,00 mg, H₃BO₃ 22,00 mg, Nasilicate 4,00 mg, NaF 2,40 mg, (NH₄)NO₃ 1,60 mg, Na₂HPO₄ 8,00 mg. Avantageusement, 37,4 g de ce mélange sont ajoutés à 1L d'eau osmosée pour obtenir 1L de milieu liquide (le site http://www.bd.com/ds/technicalCenter/inserts/difcoBblManual.asp décrit la procédure de préparation du milieu). Il peut encore s'agir d'une gélose marine, qui contient des ingrédients d'eau de mer synthétique (NaCl, MgCl₂, NaSO₄, CaCl₂, KCl, etc.) ainsi que de la peptone et de l'extrait de levure en tant que source d'azote organique, de vitamines et de minéraux. Les conditions appropriées pour la mise en culture de la population bactérienne selon l'invention incluent la température, avantageusement entre 15 et 20°C, le temps de culture, avantageusement de 24 à 36 heures, et sous agitation, avantageusement entre 200 et 250 rpm.

De préférence, le milieu de culture de l'étape (a) contient en outre un carraghénane, ceci afin d'induire une activité 4S-iota-carraghénane sulfatase. De manière encore plus préférée, le carraghénane est choisi parmi le iota-carraghénane, le lambda-carraghénane et le nu-carraghénane.

De manière préférée entre toutes, lorsque la population bactérienne est une population bactérienne *Pseudoalteromonas carrageenovora,* le milieu de culture de l'étape (a) contient en outre du lambda-carraghénane, ceci afin d'induire une activité 4S-iota-carraghénane sulfatase.

Selon une autre alternative, lorsque la population bactérienne est une population bactérienne *Pseudoalteromonas haloplanktis,* le milieu de culture de l'étape (a) contient en outre du iota-carraghénane ou un mélange iota/nu-carraghénane, ceci afin d'induire une activité 4S-iota-carraghénane sulfatase.

Avantageusement, la concentration en carraghénane ajouté au milieu de culture à l'étape (a) est comprise entre 0,5 et 1,5 g/L, de préférence 1,0 g/L. De manière encore plus avantageuse, la concentration en lambda-carraghénane ajouté au milieu de culture à l'étape (a) est comprise entre 0,5 et 1,5 g/L, de préférence 1,0 g/L.

De préférence, le procédé d'obtention selon l'invention est tel que l'étape (b) inclut les sous-étapes suivantes :
(b1) la centrifugation du milieu de culture obtenu à l'étape (a), et
(b2) l'obtention de la 4S-iota-carraghénane sulfatase à partir du surnageant et/ou du culot de culture obtenu à l'étape (b1), avantageusement à partir du culot de culture.

Pour obtenir la 4S-iota-carraghénane sulfatase à partir du surnageant, ledit surnageant est de préférence concentré, par exemple par précipitation à saturation en sulfate d'ammonium.

Pour obtenir la 4S-iota-carraghénane sulfatase à partir du culot, ledit culot est de préférence remis en suspension dans un tampon approprié tel que du tampon Tris HCL, et les cellules sont ensuite lysées par toute technique appropriée, par exemple en utilisant la presse de French. Une centrifugation est réalisée en dernier lieu pour éliminer les débris cellulaires.

De manière préférée entre toutes, le procédé d'obtention d'une 4S-iota-carraghénane sulfatase à partir d'une population bactérienne *Pseudoalteromonans carrageenovora* comprend :
(a) la mise en culture de la population bactérienne *Pseudoalteromonas carrageenovora* dans un milieu et conditions de culture appropriés pour l'obtention de la 4S-iota-carraghénane sulfatase dans le milieu de culture, ledit milieu contenant en outre du lambda-carraghénane,
(b1) la centrifugation du milieu de culture obtenu à l'étape (a), et
(b2) l'obtention de la 4S-iota-carraghénane sulfatase à partir du culot de culture obtenu à l'étape (b1).

Selon une autre alternative préférée entre toutes, le procédé d'obtention d'une 4S-iota-carraghénane sulfatase à partir d'une population bactérienne *Pseudoalteromonans haloplanktis* comprend :
(a) la mise en culture de la population bactérienne *Pseudoalteromonas haloplanktis* dans un milieu et conditions de culture appropriés pour l'obtention de la 4S-iota-carraghénane sulfatase dans le milieu de culture, ledit milieu contenant en outre du iota-carraghénane ou un mélange iota/nu-carraghénane,
(b1) la centrifugation du milieu de culture obtenu à l'étape (a), et
(b2) l'obtention de la 4S-iota-carraghénane sulfatase à partir du culot de culture obtenu à l'étape (b1).

L'étape (b) d'obtention de la 4S-iota-carraghénane sulfatase à partir du milieu de culture peut être réalisée selon différentes techniques de purification bien connues de l'homme de l'art. Il en va de même pour l'étape (b2) d'obtention de la 4S-iota-carraghénane sulfatase à partir du surnageant et/ou du culot de culture. Il s'agit en général de purifier la protéine, en l'occurrence la 4S-iota-carraghénane sulfatase. Les techniques de purification les plus couramment utilisées font appel à des méthodes chromatographiques, telle que la chromatographie d'interactions hydrophobes (par exemple sur colonne de phényle sépharose), et/ou la chromatographie sur colonne échangeuse d'anions. De préférence, les méthodes chromatographiques sont mises en oeuvre successivement. De manière encore plus préférée, on utilise d'abord la chromatographie d'interactions hydrophobes puis la chromatographie sur colonne échangeuse d'anions. Souvent, entre ces étapes successives de purification, il convient d'éliminer les sels ou produits utilisés dans les chromatographies. On utilise pour ce faire une dialyse et/ou une ultrafiltration.

De préférence, après la chromatographie d'interactions hydrophobes, les différentes fractions collectées sont dialysées puis testées pour leur capacité à désulfater le iota-carraghénane (dosage de l'activité sulfatase). Les fractions capables de désulfater le iota-carraghénane sont rassemblées et dialysées, puis l'échantillon obtenu est purifié sur une colonne échangeuse d'anions. Puis les différentes fractions collectées sont testées pour leur capacité à désulfater le iota-carraghénane (dosage de l'activité sulfatase). On peut également analyser le degré de pureté des différentes fractions par électrophorèse sur gel de polyacrylamide, la bande correspondant à la 4S-iota-carraghénane sulfatase étant alors de 110 kDaltons environ.

De préférence, le procédé d'obtention de la 4S-iota-carraghénane sulfatase selon la présente invention comprend, après l'étape (b) (ou l'étape b2), l'étape additionnelle suivante :
(c) le dosage de l'activité de la 4S-iota-carraghénane sulfatase.

Le dosage de l'activité de la 4S-iota-carraghénane sulfatase est réalisé par dosage du sulfate libéré. Pour cela, l'échantillon à doser (différentes fractions séparées ou rassemblées) est incubé avec le carraghénane, avantageusement le iota-carraghénane, de préférence à une concentration 1,2 %, de préférence volume à volume. Après un temps d'incubation minimum nécessaire, avantageusement au moins 15 heures, et à une température comprise entre 25 et 37°C, le mélange réactionnel est de préférence dilué, puis est centrifugé (microcons 10, Amicon). Puis la quantité de sulfate présente dans le filtrat est avantageusement dosée par chromatographie haute performance échangeuse d'anions. En dernier lieu, la détection des anions peut être réalisée par conductimétrie. Pour cela, on utilisera de préférence un détecteur du type ED40 (Dionex) muni d'un suppresseur ASRSD ultra-II-4 mm.

Selon un autre aspect, la présente invention a pour objet un procédé d'extraction d'une 4S-iota-carraghénane sulfatase à partir d'une population d'algues rouges marines, comprenant :
(i) l'isolement d'une population bactérienne susceptible de contenir la 4S-iota-carraghénane sulfatase à partir de la population d'algues rouges marines ; et
(ii) l'obtention de la 4S-iota-carraghénane sulfatase à partir de la population bactérienne obtenue à l'étape (i), selon le procédé tel que défmi ci-dessus.

L'isolement d'une population bactérienne, telle qu'une population bactérienne susceptible de contenir la 4S-iota-carraghénane sulfatase, à partir d'une population d'algues marines est bien connue de l'homme de l'art et est notamment décrite dans *Potin et al., 1991.*

Selon encore un autre aspect, la présente invention a pour objet un procédé de conversion d'une composition contenant du iota-carraghénane et/ou du iota-carraghénane hybride, en une composition qui contient de l'alpha-carraghénane et/ou de l'alpha-carraghénane hybride, comprenant les étapes suivantes :
(a) la mise en présence de la composition contenant du iota-carraghénane et/ou du iota-carraghénane hybride, avec une solution contenant un polypeptide isolé comprenant une séquence amino-acide ayant au moins 65 % d'identité avec la séquence SEQ ID N°2 ou la séquence SEQ ID N°4, dans des conditions appropriées pour la conversion de la composition, et
(b) le cas échéant, la récupération de la composition contenant l'alpha-carraghénane et/ou l'alpha-carraghénane hybride obtenue à l'étape (a).

Le polypeptide isolé comprenant une séquence amino-acide ayant au moins 65 % d'identité avec la séquence SEQ ID N°2 ou la séquence SEQ ID N°4 ainsi que ses modes de réalisation préférés sont tels que définis ci-dessus.

Dans la présente invention, on entend par « composition contenant du iota-carraghénane et/ou du iota-carraghénane hybride » une solution, une solution partiellement gélifiée ou un gel contenant ledit iota-carraghénane et/ou iota-carraghénane hybride. En effet, il convient de noter que, dans des conditions expérimentales telles que celles de l'invention, le iota-carraghénane pur gélifie sous ses propres charges. Ainsi dans les conditions expérimentales, on ne peut pas parler *stricto sensu* de solution. De plus, dans des conditions très diluées, le iota-carraghénane ne gélifie pas. Toutefois, des solutions peuvent être obtenues avec du iota-carraghénane pour des températures plus élevées, c'est-à-dire en général supérieures à 40°C. Par ailleurs, les propriétés de gélification sont modulées par la structure des carraghénanes hybrides. Ainsi, par exemple, le iota-nu-carraghénane étant peu gélifiant, on pourra l'avoir plus facilement sous forme de solution. Il en va de même pour les oligo-iota-carraghénanes.

Dans la présente invention, on entend par « iota-carraghénane » un composé constitué d'au moins un motif iota-carrabiose (n = 1) (cf. schéma 1 ci-dessus). De préférence, il s'agira d'un oligo-iota-carraghénane (1 ≤ n ≤ 20), voire d'un polymère de iota-carraghénane (n ≥ 20).

Egalement, on entend par « iota-carraghénane hybride » un composé constitué d'au moins un motif iota-carrabiose (n = 1) et d'au moins un motif carrabiose autre que iota-carrabiose (m = 1), tel que, sans toutefois s'y limiter, un motif nu-carrabiose, kappa-carrabiose, mu-carrabiose ou alpha-carrabiose. De préférence, il s'agira d'un oligo-iota-carraghénane hybride (1 ≤ n+m ≤ 20), voire d'un polymère de iota-carraghénane hybride (n+m ≥ 20) (copolymère).

Egalement dans la présente invention, on entend par « composition contenant de l'alpha-carraghénane et/ou de l'alpha-carraghénane hybride » une composition qui contient un iota-carraghénane ou un iota-carraghénane hybride dans lequel au moins un motif iota-carrabiose est converti en motif alpha-carrabiose. La conversion, au moins partielle, ou totale, de la composition contenant du iota-carraghénane et/ou du iota-carraghénane hybride en une composition contenant de l'alpha-carraghénane et/ou de l'alpha-carraghénane hybride, dépend des conditions expérimentales, c'est-à-dire de la concentration d'enzyme, de substrat, la température, et du temps de mise en présence avec l'enzyme. De préférence, l'ensemble des motifs iota-carrabiose présents dans le iota-carraghénane et/ou le iota-carraghénane hybride sont convertis en motifs alpha-carrabiose.

L'étape (b) est facultative. En effet, pour une analyse expérimentale, il n'est pas nécessaire de séparer la solution contenant la 4S-iota-carraghénane-sulfatase de la composition obtenue contenant l'alpha-carraghénane et/ou l'alpha-carraghénane hybride. Par contre, cette séparation serait nécessaire dans d'autres applications comme en biomédicale, auquel cas on pourrait par exemple utiliser l'ultra-filtration.

Avantageusement, à l'étape (a), on ajoute de la solution contenant le polypeptide dans le milieu réactionnel afin d'augmenter le taux de conversion.

Les conditions appropriées pour la conversion de la composition contenant du iota-carraghénane et/ou du iota-carraghénane hybride (substrat) en une composition contenant de l'alpha-carraghénane et/ou de l'alpha-carraghénane hybride incluent la température, avantageusement entre 25 et 37°C, le temps de mise en présence de la composition contenant le substrat avec la solution contenant l'enzyme, avantageusement de 12 à 25 heures, et la concentration en enzyme et en substrat. De préférence, on mélange l'enzyme et le substrat volume à volume.

La présente invention a également pour objet un procédé de conversion d'une composition contenant du iota-carraghénane et/ou du iota-carraghénane hybride présent dans une population d'algues rouges, en une composition qui contient de l'alpha-carraghénane et/ou de l'alpha-carraghénane hybride, comprenant les étapes suivantes :
(a) la mise en présence de la population d'algues rouges avec une solution contenant un polypeptide isolé comprenant une séquence amino-acide ayant au moins 65 % d'identité avec la séquence SEQ ID N°2 ou la séquence SEQ ID N°4, dans des conditions appropriées pour la conversion de la composition contenant le iota-carraghénane et/ou le iota-carraghénane hybride présent dans une population d'algues rouges, et
(b) le cas échéant, la récupération de la composition contenant l'alpha-carraghénane et/ou l'alpha-carraghénane hybride obtenue à l'étape (a).

De préférence, la composition contenant du iota-carraghénane et/ou du iota-carraghénane hybride est mise en présence avec une solution contenant un polypeptide isolé comprenant ou consistant en la séquence amino-acide choisie dans le groupe constitué par la séquence SEQ ID N°2 et la séquence SEQ ID N°4.

La composition contenant du iota-carraghénane et/ou du iota-carraghénane hybride peut être obtenue à partir d'algues rouges marines, ou bien dans le commerce, notamment auprès de CP Kelco, Cargill, ou auprès de Sigma.

L'invention a également pour objet l'utilisation d'un polypeptide isolé comprenant une séquence amino-acide ayant au moins 65 % d'identité avec la séquence SEQ ID N°2 ou la séquence SEQ ID N°4, pour la préparation d'un agent de texture comprenant de l'alpha-carraghénane et/ou de l'alpha-carraghénane hybride. L'agent de texture peut être utilisé en industrie agro-alimentaire, parapharmacie et cosmétique.

L'invention a également pour objet l'utilisation d'un polypeptide isolé comprenant une séquence amino-acide ayant au moins 65 % d'identité avec la séquence SEQ ID N°2 ou la séquence SEQ ID N°4, pour la préparation d'un agent gélifiant comprenant de l'alpha-carraghénane et/ou de l'alpha-carraghénane hybride.

L'invention a encore pour objet l'utilisation d'un polypeptide isolé comprenant une séquence amino-acide ayant au moins 65 % d'identité avec la séquence SEQ ID N°2 ou la séquence SEQ ID N°4, pour la préparation d'un agent épaississant comprenant de l'alpha-carraghénane et/ou de l'alpha-carraghénane hybride.

Selon un autre aspect, la présente invention a pour objet l'utilisation d'un polypeptide isolé comprenant une séquence amino-acide ayant au moins 65 % d'identité avec la séquence SEQ ID N°2 ou la séquence SEQ ID N°4, pour la conversion partielle ou totale d'une composition contenant du iota-carraghénane et/ou du iota-carraghénane hybride, en une composition qui contient de l'alpha-carraghénane et/ou de l'alpha-carraghénane hybride.

De préférence, la séquence amino-acide est choisie dans le groupe constitué par la séquence SEQ ID N°2 et la séquence SEQ ID N°4.

Egalement de manière préférée, la composition contenant du iota-carraghénane et/ou du iota-carraghénane hybride est obtenue à partir d'algues rouges marines.

La présente invention a pour objet un polypeptide isolé comprenant ou consistant en une séquence amino-acide ayant au moins 65 % d'identité avec la séquence SEQ ID N°2 ou la séquence SEQ ID N°4, à l'exclusion du polypeptide de séquence SEQ ID N°5.

Les exemples et figures qui suivent permettent d'illustrer la présente invention sans toutefois en limiter la portée.

### LEGENDES DES FIGURES

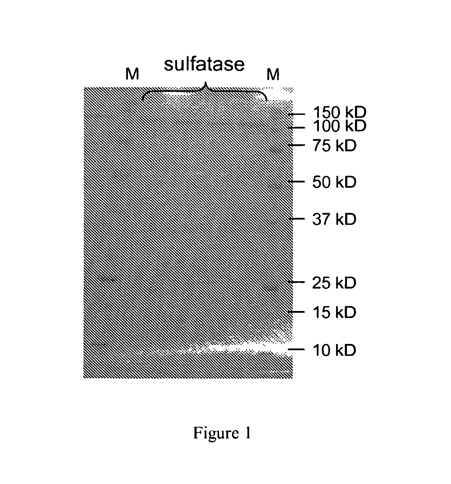

**Figure 1** **:** SDS-PAGE (12%). Le marqueur de taille (M) est un mélange commercial (BioRad Precision) de 9 protéines caractérisées par des poids moléculaires compris entre 250 et 10kD.

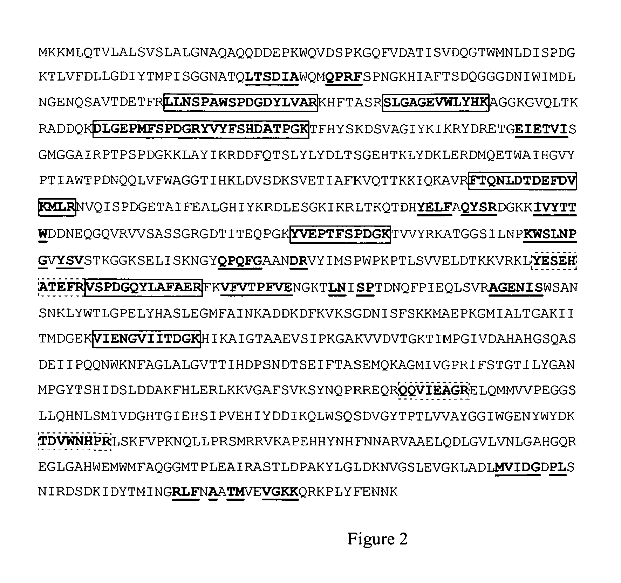

**Figure 2****:** Structure primaire de la protéine hypothétique Q3IKL4 de *Pseudoalteromonans haloplanktis.* En gras encadré trait plein ou gras souligné les peptides déduits des expériences des LC/MS-MS (gras et encadré trait plein) ou de *de novo* (gras et souligné). En gras et encadré trait pointillé, les peptides prédits par les expériences de MALDI.

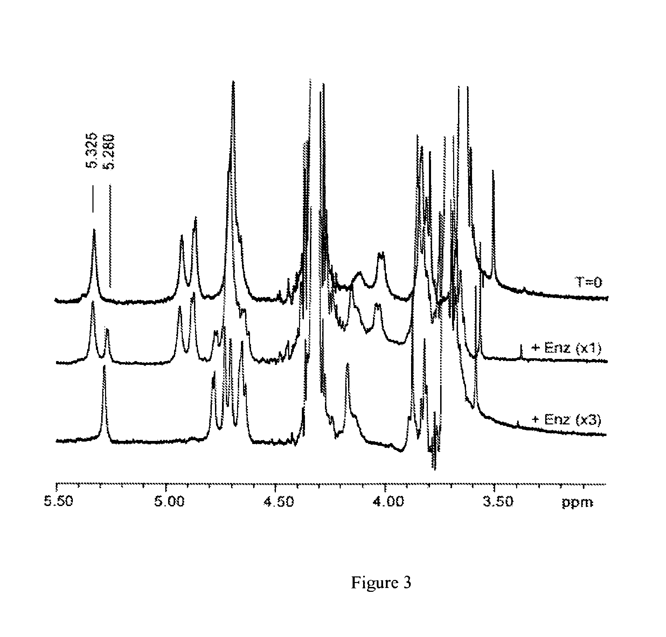

**Figure 3** **:** Spectres RMN du proton enregistrés sur du iota-carraghénane incubé avec la 4S-iota-caraghénane sulfatase purifiée qui conduit à la formation de l'alpha-carraghénane. Courbe « T=0 » : iota-carraghénane avant ajout de l'enzyme ; courbe « + Enz (*1) » : après 15 heures d'incubation à 35°C ; courbe « + Enz (*3) » : après 96 heures d'incubation à 35°C et addition d'un volume supplémentaire d'enzyme.

### EXEMPLES

### Exemple 1 : Introduction

L'objectif des Inventeurs a été d'identifier et de purifier une sulfatase capable de convertir partiellement ou complètement le iota-carraghénane en alpha-carraghénane.

Pour parvenir à cette fin, les Inventeurs ont, dans un premier temps, développé un protocole pour cultiver la souche bactérienne *Pseudoalteromonans carrageenovora 9* (ATCC 43555). Cette bactérie marine est en effet connue pour dégrader certains polysaccharides extraits des algues rouges : les carraghénanes. Plusieurs carraghénane-hydrolases, ou carraghénases, issues de cette bactérie ont été identifiées et caractérisées au laboratoire. Après un criblage de l'activité sulfatase dans différentes conditions expérimentales, les Inventeurs ont ensuite développé un protocole pour purifier à l'homogénéité l'enzyme capable de désulfater le iota-carraghénane en alpha-carraghénane.

### Exemple 2 : Matériel et méthodes

### 2.1 Dosage et identification de l'activité sulfatase :

### a) Réaction de désulfatation

1 volume d'échantillon (200 à 500µl) à doser est incubé avec 1 volume de iota-carraghénane (CP Kelco, Cargill) à une concentration de 1,4 % dans de l'eau. Pour chaque réaction enzymatique, un blanc est réalisé dans des conditions similaires mais en utilisant l'extrait enzymatique préalablement inactivé par ébullition durant minimum 10 minutes à 100°C.

Après 15 à 20 d'heures d'incubation à une température comprise entre 25 et 37°C, le mélange réactionnel est dilué 2 fois dans de l'eau milliQ (Millipore) avant d'être centrifugé dans des microcons 10 (Amicon). La centrifugation est réalisée à 3300g pendant 60 à 90 minutes à une température comprise entre 20 et 40°C.

### b) Dosage du sulfate libéré

La quantité de sulfate présente dans le filtrat est alors dosée par chromatographie échangeuse d'anions (HPAEC : high performance anion exchange chromatography) à l'aide d'un système Dionex DX 500 (Dionex Corporation, Sunnyvale, CA, USA). La séparation des anions présents dans l'échantillon est réalisée grâce à une colonne de type Ion-Pac AS11 (4 x 200 mm ; Dionex) équipée d'une pré-colonne de type AG-11 (4 x 50 mm, Dionex). Colonne et précolonne sont préalablement équilibrées dans 12 mM NaOH. L'élution des anions est réalisée à un débit de 1ml/min (pompe GP40, Dionex). La détection des anions est réalisée par conductimétrie avec un détecteur ED40 (Dionex) muni d'un suppresseur ASRSD ultra-II- 4 mm (Dionex) fonctionnant sous un courant de 100 mA.

### c) Analyse par RMN du proton

L'identification du produit formé au cours de la réaction de désulfatation est réalisée par RMN. Dans ce cas, la réaction de désulfatation est réalisée en incubant 1 ml d'échantillon avec 500 µl d'une solution de iota-carraghénane (X-6908 ; CP-Kelco) à 1,2 % dans l'eau. Le mélange est incubé pendant au moins 16 heures à 35°C. Les blancs sont réalisés de manière similaire mais avec des échantillons inactivés 30 minutes à 100°C avant l'incubation à 35°C. Après incubation, les échantillons sont lyophilisés puis échangés dans de l'eau deutérée (D₂O, 2 cycles échange/lyophilisation) avant d'être analysés en RMN (Service de RMN de l'Université de Bretagne Occidentale, Brest, N. Kervarec) (voir point **3.2.c)** ci-dessous).

### 2.2 Culture bactérienne :

Pour un criblage préliminaire de l'activité sulfatase, la souche bactérienne *Pseudoalteromonas carrageenovora* 9 de l'American Type Culture Collection (ATCC 43555) a été ensemencée dans du milieu Zobell.

La composition du milieu Zobell est la suivante :

| | |
|---|---|
| Bacto peptone (Difco) | 5g |
| Extrait de levure (Difco) | 1g |
| Eau de mer filtrée | 800 ml |
| Eau osmosée | 200 ml |

Afin d'induire potentiellement une activité sulfatase, les cultures bactériennes (5x 1000 ml) sont réalisées en présence de différents carraghénanes (1g/l) dans un agitateur de type New Brunswick sous une agitation comprise entre 150 et 200 rpm.

Après 36-40 heures d'incubation à une température comprise entre 15 et 20°C, le milieu de culture est centrifugé entre 7500 et 9800 g pendant 30-60 minutes. L'activité enzymatique est mesurée sur le surnageant et sur le culot de culture. Avant de tester son activité, le surnageant de culture est préalablement concentré par ultrafiltration sur une membrane dont le seuil d'exclusion est de 10 kD. Le filtrat obtenu dont le volume est d'environ 200 à 300 ml est davantage concentré par précipitation à saturation en sulfate d'ammonium (65g de sulfate d'ammonium/100 ml d'échantillon).Après centrifugation, le culot obtenu est remis en suspension dans un volume minimum de tampon 50 mM Tris HCL pH 8.3 et dialysé au moins 72 heures contre ce tampon.

Le culot de culture est remis en suspension pendant environ 1 heure dans 30 à 50 ml de tampon 50 mM Tris (Sigma) HCl pH 8.3. Les cellules sont ensuite lysées à l'aide d'une presse de French. Une centrifugation à 25- 29000g pendant 40 à 60 minutes est réalisée pour éliminer les débris cellulaires.

Pour la **production de 4S-carraghénane sulfatases,** les cultures (5X 1L) sont réalisées dans des conditions similaires mais l'induction de la production de sulfatase est réalisée en présence de lambda carraghénane (1g/l) (voir point **3.1** ci-dessous).

### 2.3 Purification de la sulfatase

Sauf indications contraires, toutes les étapes décrites dans ce paragraphe sont réalisées à 4°C.

En fin de culture, le milieu de culture est centrifugé pendant 30-60 minutes entre 7500 et 9800 g. Le culot obtenu est remis en suspension dans 30 à 50 ml de tampon 50 mM Tris (Sigma) HCl pH 8.3. Une pastille de Complete EDTA-free (Roche) est ajoutée. Les cellules sont lysées avec une presse de French (Aminco). Après centrifugation, le surnageant ainsi obtenu est ensuite amené à 30% de saturation (16,4 g/100 ml) de sulfate d'ammonium.

Après une centrifugation (25 à 29000g ; 40 à 60 min.), le surnageant est ensuite déposé sur une résine de Phényle sépharose 6 FF high sub (GE Healthcare) (19.5x 2 cm) préalablement équilibrée dans du tampon 50 mM Tris HCl (pH 8.3) saturé à 30% de sulfate d'ammonium (16.4g/100ml). La résine est lavée avec ce tampon jusqu'à ce que l'absorbance à 280 nm soit négligeable. Les protéines sont ensuite éluées avec un gradient décroissant de sulfate d'ammonium. En 20 volumes de colonne, on passe de 30 % à 0 % de saturation en sulfate d'ammonium. Les fractions (6,5 ml) capables de désulfater le iota-carraghénane sont rassemblées et dialysées à l'aide de membranes SpectraPor dont le cut-off est de 3500 daltons. La dialyse est effectuée dans du tampon 50 mM tris HCl (pH 8,3).

L'échantillon est ensuite déposé sur une résine échangeuse d'anions de type DEAE Sépharose FF (GE Healthcare) (13x 1.6 cm) préalablement équilibrée dans du tampon 50 mM Tris HCl (pH8.3). Après lavage de la résine avec ce même tampon, les protéines sont éluées avec un gradient croissant de NaCl. La concentration finale de 1M en NaCl est atteinte en 20 volumes de colonne. Les différentes fractions collectées durant l'élution (vol. : 5.5 ml) sont testées pour leur capacité à désulfater le iota carraghénane. Le degré de pureté des différentes fractions est également analysé par électrophorèse sur gel de polyacrylamide (SDS-PAGE) (Laemmli and Favre, (1973) J. Biol. Chem.. 80, 575- 599).

### Exemple 3 : Résultats et discussion

### 3.1 Culture bactérienne :

### Criblage de l'activité sulfatase

Comme le montre le tableau 3 ci-dessous, la présence de iota-, de lambda- ou de nu-carraghénane dans le milieu de culture induit à des degrés divers la production de sulfatase active sur le iota- et sur le nu-carraghénane.

**Tableau 3 : Activité sulfatase dans les surnageants et les culots de culture après induction avec du iota, du nu ou du lambda carraghénane.**

| | **Activité sulfatase sur le** | |
|---|---|---|
| Culture induite avec du | Iota-carraghénane | Iota/Nu-carraghénane |
| **Iota-carraghénane** | | |
| Surnageant de culture | + | - |
| Culot de culture | + | + |

| **Lambda-carrashénane** | | |
|---|---|---|
| Surnageant de culture | - | - |
| Culot de culture | +++ | ++ |

| **Nu-carraghénane** | | |
|---|---|---|
| Surnageant de culture | ++ | - |
| Culot de culture | ++ | ++ |

Pour une production optimale d'enzyme, on peut constater que c'est le lambda carraghénane qui s'avère le plus efficace. En effet, l'activité observée avec ce carraghénane est environ 3 fois supérieure à celle observée lorsqu'on utilise le iota-carraghénane comme inducteur.

### 3.2 Purification et caractérisation de la 4S-iota-sulfatase

### a) Purification

La chromatographie d'interactions hydrophobes (i.e. phényle sépharose) est une étape importante car elle nous permet d'éliminer une grande partie des autres protéines présentes dans le culot de bactéries. En effet, la sulfatase élue entre 17 et 15 % de saturation en sulfate d'ammonium tandis que les autres protéines éluent lorsque la concentration en sulfate d'ammonium est inférieure à ces valeurs, voire nulle.

Toutes les fractions qui présentent une activité sulfatase sur le iota carraghénane sont rassemblées et dialysées pendant au moins 72 heures. L'échantillon dialysé (environ 140 ml) est ensuite purifié par chromatographie sur une résine échangeuse d'anions. La mesure de l'activité enzymatique des différentes fractions récoltées (80) montre que la sulfatase élue lorsque la concentration en NaCl est comprise entre 300 et 430 mM. Le maximum d'activité sulfatase est en fait observé dans les fractions sulfatases qui éluent avec une concentration de NaCl comprise entre 340 et 370 mM. L'analyse électrophorétique en conditions dénaturantes de ces fractions montre que la 4S-iota-cararghénane sulfatase se présente sous forme d'une protéine unique dont le poids moléculaire est d'environ 110 kD (voir Figure 1).

### b) Détermination de la séquence peptidique

Afin de déterminer la séquence peptidique de la sulfatase, la bande correspondant à la protéine a été découpée et soumise à une digestion trypsique. Les peptides obtenus ont été purifiés et analysés par spectrométrie de masse sur la plate-forme RIO "Biopolymères" localisée à l'INRA de Nantes. Les données LC-MS/MS confrontées à la banque TrEMBL ont montré une très grande homologie de séquence avec la protéine Q3IKL4 de *P. haloplanktis* (voir Figure 2). Cette séquence a ensuite été utilisée pour créer une banque à laquelle ont été confrontées les données MALDI : La plupart des peptides majeurs (sauf 5) du spectre MALDI ont été identifiés dans cette séquence. Les peptides mesurés en LC-MS/MS et non identifiés dans la séquence Q3IKL4 ont été séquencés par spectrométrie de masse (*"de novo") :* tous ces fragments correspondent à des morceaux de séquence que l'on retrouve dans Q3IKL4 (avec quelques différences qui expliquent pourquoi ces peptides n'ont pas été identifiés sur la base des informations masse+séquence).

La séquence protéique Q3IKL4 (SEQ ID N°5) correspond à une protéine hypothétique de la bactérie marine *Pseudoalteromonas haloplanktis* dont le génome a été entièrement séquencé. *P. haloplanktis* est phylogénétiquement proche de P. *carrageenovora* ce qui suppose que ces deux bactéries partagent des activités enzymatiques et des gènes en commun.

### c) Analyse du produit de la réaction de désulfatation

Voir le protocole au point 2.1.c) ci-dessus

La figure 3 montre le spectre du proton enregistré sur du iota-carraghénane avant (A) et après incubation (B et C) avec la sulfatase. Comme le montre cette figure, l'enzyme est capable de convertir progressivement le iota-carraghénane (caractérisé par un déplacement chimique à 5.32 ppm - courbe A de la figure 3) en alpha-carraghénane (caractérisé par un déplacement chimique à 5.25 ppm). La conversion complète (courbe C de la figure 3) est cependant très lente (96 heures) et nécessite l'addition d'un volume d'enzyme. La technique de RMN 2D (HMQC) a permis de confirmer que le produit formé après 96 heures d'incubation était de l'alpha-carraghénane.

### REFERENCES BIBLIOGRAPHIQUES

Falshaw et al, 1996, Carbohydrate research, 285, 82-98
Michel et al, Appl Microbiol Biotechnol. 2006 Jun;71(1):23-33. Epub 2006 Mar 21. Review
Potin et al, Eur J Biochem. 1991 Oct 1;201(1):241-7.
Zablackis et Santos, 1986, Botanica marina, 29, 319-322

## Revendications

1. Polypeptide isolé comprenant une séquence amino-acide ayant au moins 95 % d'identité avec une séquence amino-acide choisie dans le groupe constitué par les séquences SEQ ID N°2 et SEQ ID N°4, ledit polypeptide présentant une activité 4S-iota-carraghénane sulfatase.

2. Polynucléotide isolé choisi parmi les polynucléotides suivants :
a) un polynucléotide, ADN ou ARN, codant le polypeptide selon la revendication 1 ; et
b) un polynucléotide complémentaire d'un polynucléotide tel que défini en a).

3. Polynucléotide selon la revendication 2, qui code le polypeptide selon la revendication 1 et comprend une séquence nucléotidique ayant au moins 65 % d'identité avec la séquence SEQ ID N°1 ou la séquence SEQ ID N°3.

4. Polynucléotide selon la revendication 3, dans lequel la séquence nucléotidique est choisie dans le groupe constitué par la séquence SEQ ID N°1 et la séquence SEQ ID N°3.

5. Vecteur comprenant un polynucléotide selon l'une des revendications 2 à 4.

6. Cellule hôte comprenant un vecteur selon la revendication 5.

7. Procédé de production d'un polypeptide selon la revendication 1 comprenant les étapes suivantes :
a) la culture dans un milieu et conditions de culture appropriés d'une population de cellules hôtes selon la revendication 6 ; et
b) la récupération dudit polypeptide ainsi produit à partir du milieu de culture ou de la population de cellules hôtes cultivées.

8. Procédé d'obtention d'une 4S-iota-carraghénane sulfatase à partir d'une population bactérienne contenant une 4S-iota-carraghénane sulfatase, ledit procédé comprenant :
(a) la mise en culture d'une population bactérienne dans un milieu contenant du carraghénane et conditions de culture appropriés pour l'obtention de la 4S-iota-carraghénane sulfatase dans le milieu de culture, ladite population bactérienne étant une population bactérienne *Pseudoalteromonas,* avantageusement une population bactérienne *Pseudoalteromonas haloplanktis* et/ou *Pseudoalteromonas carrageenovora,* et
(b) l'obtention d'une 4S-iota-carraghénane sulfatase à partir du milieu de culture obtenu à l'étape (a).

9. Procédé d'obtention selon la revendication 8 dans lequel à l'étape (a), lorsque la population bactérienne est une population bactérienne *Pseudoalteromonas carrageenovora,* le milieu de culture contient du lambda-carraghénane.

10. Procédé d'obtention selon la revendication 8 dans lequel à l'étape (a), lorsque la population bactérienne est une population bactérienne *Pseudoalteromonas haloplanktis,* le milieu de culture contient du iota-carraghénane et/ou un mélange de iota-/nucarraghénane.

11. Procédé d'obtention selon la revendication 9, dans lequel l'étape (b) inclut les sous-étapes suivantes :
(b1) la centrifugation du milieu de culture obtenu à l'étape (a), et
(b2) l'obtention de la 4S-iota-carraghénane sulfatase à partir du culot de culture obtenu à l'étape (b1).

12. Procédé d'obtention selon l'une des revendications 8 à 11 qui comprend, après l'étape (b), l'étape additionnelle suivante :
(c) le dosage de l'activité de la 4S-iota-carraghénane sulfatase.

13. Procédé d'extraction d'une 4S-iota-carraghénane sulfatase à partir d'une population d'algues rouges marines, comprenant :
(i) l'isolement d'une population bactérienne contenant une 4S-iota-carraghénane sulfatase à partir de la population d'algues rouges marines, ladite population bactérienne étant une population bactérienne *Pseudoalteromonas,* avantageusement une population bactérienne *Pseudoalteromonas haloplanktis* et/ou *Pseudoalteromonas carrageenovora;* et
(ii) l'obtention d'une 4S-iota-carraghénane sulfatase à partir de la population bactérienne obtenue à l'étape (i), selon le procédé défini dans l'une des revendications 8 à 12.

14. Procédé de conversion d'une composition contenant du iota-carraghénane et/ou du iota-carraghénane hybride, en une composition qui contient de l'alpha-carraghénane et/ou de l'alpha-carraghénane hybride, comprenant les étapes suivantes :
(a) la mise en présence de la composition contenant du iota-carraghénane et/ou du iota-carraghénane hybride, avec une solution contenant un polypeptide isolé comprenant une séquence amino-acide ayant au moins 65 % d'identité avec la séquence SEQ ID N°2 ou la séquence SEQ ID N°4 et présentant une activité 4S-iota-carraghénane sulfatase, dans des conditions appropriées pour la conversion de la composition, et
(b) le cas échéant, la récupération de la composition contenant l'alpha-carraghénane et/ou l'alpha-carraghénane hybride obtenue à l'étape (a).

15. Procédé de conversion selon la revendication 14, dans lequel, à l'étape (a), on ajoute de la solution contenant le polypeptide dans le milieu réactionnel afin d'augmenter le taux de conversion.

16. Procédé selon la revendication 14 ou 15, dans lequel la séquence amino-acide est choisie dans le groupe constitué par la séquence SEQ ID N°2 et la séquence SEQ ID N°4.

17. Procédé selon l'une des revendications 14 à 16, dans lequel la composition contenant du iota-carraghénane et/ou du iota-carraghénane hybride est obtenue à partir d'algues rouges marines.

18. Utilisation d'un polypeptide isolé comprenant une séquence amino-acide ayant au moins 65 % d'identité avec la séquence SEQ ID N°2 ou la séquence SEQ ID N°4 et présentant une activité 4S-iota-carraghénane sulfatase, pour la préparation d'un agent de texture comprenant de l'alpha-carraghénane et/ou de l'alpha-carraghénane hybride.

## Patentansprüche

1. Isoliertes Polypeptid umfassend eine Aminosäuresequenz mit zumindest 95% Identität mit einer Aminosäuresequenz, ausgewählt aus der Gruppe bestehend aus den Sequenzen SEQ ID N°2 und SEQ ID N°4, wobei das Polypeptid eine 4S-Iota-Carrageen-Sulfatase-Aktivität aufweist.

2. Isoliertes Polynukleotid, ausgewählt aus den folgenden Polynukleotiden:
a) ein Polynukleotid, DNA oder RNA, das das Polypeptid nach Anspruch 1 kodiert ; und
b) ein zu einem Polynukleotid komplementar Polynukleotid, das komplementar ist zu einem Polynukleotid wie unter a) definiert.

3. Polynukleotid nach Anspruch 2, das das Polypeptid nach Anspruch 1 kodiert und eine Nukleotidsequenz mit zumindest 65 % Identität mit der Sequenz SEQ ID N°1 oder der Sequenz SEQ ID N°3 aufweist.

4. Polynukleotid nach Anspruch 3, in welchem die Nukleotidsequenz ausgewählt ist aus der Gruppe bestehend aus der Sequenz SEQ ID N°1 und der Sequenz SEQ ID N°3.

5. Vektor umfassend ein Polynukleotid nach einem der Ansprüche 2 bis 4.

6. Wirtszelle umfassend einen Vektor nach Anspruch 5.

7. Herstellungsverfahren eines Polypeptids nach Anspruch 1, welches die folgenden Schritte umfasst :
a) das Züchten in geeigneten Milieu- und Kulturbedingungen einer Wirtszellenpopulation nach Anspruch 6 ; und
b) die Rückgewinnung des auf diese Weise ausgehend von dem Kulturmilieu oder der gezüchteten Wirtszellenpopulation hergestellten Polypeptids.

8. Verfahren zum Erhalt einer 4S-Iota-Canageen-Sulfatase ausgehend von einer Bakterienpopulation, die eine 4S-Iota-Carrageen-Sulfatase enthält, wobei das Verfahren umfasst :
(a) das Züchten einer Bakterienpopulation in einem Carrageen enthaltenden Milieu und geeigneten Kulturbedingungen, um 4S-Iota-Carrageen-Sulfatase in dem Kulturmedium zu erhalten, wobei die Bakterienpopulation eine *Pseudoalteromonas-Bakterienpopulation,* vorzugsweise eine *Pseudoalteromonas haloplanktis* und/oder *Pseudoalteromonas carrageenovora*-Bakterienpopulation ist, und
(b) der Erhalt einer 4S-Iota-Carrageen-Sulfatase ausgehend von dem in Schritt (a) erhaltenen Kulturmedium.

9. Verfahren zum Erhalt nach Anspruch 8, in welchem in Schritt (a), wenn die Bakterienpopulation eine *Pseudoalteromonas carrageenovora*-Bakterienpopulation ist, das Kulturmedium Lambda-Carrageen enthält.

10. Verfahren zum Erhalt nach Anspruch 8, in welchem in Schritt (a), wenn die Bakterienpopulation eine *Pseudoalteromonas haloplanktis*-Bakterienpopulation ist, das Kulturmedium Iota-Carrageen und/oder eine Mischung aus Iota-/Nu-Carrageen enthält.

11. Verfahren zum Erhalt nach Anspruch 9, in welchem Schritt (b) folgende Unterschritte umfasst :
(b1) die Zentrifugierung des in Schritt (a) erhaltenen Kulturmediums, und
(b2) die Herstellung von 4S-Iota-Carrageen-Sulfatase ausgehend von dem in Schritt (b1) erhaltenen Kulturansatz.

12. Verfahren zum Erhalt nach einem der Ansprüche 8 bis 11, welches nach Schritt (b) den folgenden zusätzlichen Schritt umfasst :
(c) die Dosierung der Aktivität der 4S-Iota-Carrageen-Sulfatase.

13. Extraktionsverfahren einer 4S-Iota-Carrageen-Sulfatase ausgehend von einer Population roter Meeralgen, umfassend:
(i) die Isolierung einer Bakterienpopulation, die eine 4S-Iota-Carrageen-Sulfatase ausgehend von der Population roter Meeralgen umfasst, wobei diese Bakterienpopulation eine *Pseudoalteromonas-*Bakterienpopulation*,* vorzugsweise eine *Pseudoalteromonas haloplanktis* und/oder *eine Pseudoalteromonas carrageenovora*-Bakterienpopulation ist; und
(ii) der Erhalt einer 4S-Iota-Carrageen-Sulfatase nach einem der in den Ansprüchen 8 bis 12 definierten Verfahren aus einer in Schritt (i) erhaltenen Bakterienpopulation.

14. Umwandlungsverfahren einer Zusammensetzung enthaltend IotaCarrageen und/oder ein Iota-Carrageen-Hybrid in einer Zusammensetzung, welche Alpha-Carrageen und/oder Alpha-Carrageen-Hybrid enthält, mit den folgenden Schritten:
(a) das Inkontaktbringen der Iota-Carrageen und/oder Iota-Carrageen-Hybrid enthaltenden Zusammensetzung mit einer Lösung enthaltend ein isoliertes Polypeptid, welches eine Aminosäuresequenz enthält, die zumindest 65 % Identität mit der Sequenz SEQ ID N°2 oder der Sequenz SEQ ID N°4 aufweist und eine 4S-Iota-Carrageen-Sulfatasenaktivität aufweist, unter für die Umwandlung der Zusammensetzung geeigneten Bedingungen, und
(b) gegebenenfalls die Rückgewinnung der in Schritt (a) erhaltenen Alpha-Carrageen und/oder Alpha-Carrageen-Hybrid enthaltenden Zusammensetzung.

15. Umwandlungsverfahren nach Anspruch 14, in welchem in Schritt (a) die das Polypeptid enthaltende Lösung zum Reaktionsgemisch hinzugefügt wird, um die Umwandlungsrate zu erhöhen.

16. Verfahren nach Anspruch 14 oder 15, in welchem die Aminosäuresequenz aus der Gruppe ausgewählt ist, die aus der Sequenz SEQ ID N°2 und der Sequenz SEQ ID N°4 besteht.

17. Verfahren nach einem der Ansprüche 14 bis 16, in welchem die Iota-Carrageen und/oder Iota-Carrageen-Hybrid enthaltende Zusammensetzung aus roten Meeralgen erhalten ist.

18. Verwendung eines isolierten Polypeptids umfassend eine Aminosäuresequenz mit mindestens 65 % Identität mit der Sequenz SEQ ID N°2 oder der Sequenz SEQ ID N°4 und eine 4S-Iota-Carrageen-Sulfatase für die Herstellung eines Texturmittels aufweisend, welches Alpha-Carrageen und/oder Alpha-Carrageen-Hybrid enthält.

## Claims

1. Isolated polypeptide comprising an amino acid sequence having at least 95% identity with an amino acid sequence selected from the group consisting of sequences SEQ ID N°2 and SEQ ID N°4, said polypeptide having a 4S-iota-carrageenan sulfatase activity.

2. Isolated polynucleotide selected from the following polynucleotides:
a) a polynucleotide, DNA or RNA, encoding the polypeptide according to claim 1; and
b) a complementary polynucleotide of a polynucleotide as defined in a).

3. Polynucleotide according to claim 2, which encodes the polypeptide according to claim 1 and comprises a nucleotide sequence having at least 65% identity with the sequence SEQ ID N° 1 or the sequence SEQ ID N°3.

4. Polynucleotide according to claim 3, in which the nucleotide sequence is selected from the group consisting of the sequence SEQ ID N°1 and the sequence SEQ ID N°3.

5. Vector comprising a polynucleotide according to one of claims 2 to 4.

6. Host cell comprising a vector according to claim 5.

7. Method for producing a polypeptide according to claim 1 comprising the following steps:
a) culturing in suitable medium and culture conditions a population of host cells according to claim 6; and
b) recovering said polypeptide thus produced from the culture medium or the population of cultured host cells.

8. Method for obtaining a 4S-iota-carrageenan sulfatase from a bacterial population containing a 4S-iota-carrageenan sulfatase, said method comprising:
a) culturing a bacterial population in a medium containing carrageenan in culture conditions suitable for obtaining 4S-iota-carrageenan sulfatase in the culture medium, said bacterial population being a of *Pseudoalteromonas bacterial population,* advantageously a of *Pseudoalteromonas haloplanktis* and/or a *Pseudoalteromonas carrageenovora bacterial population,* and
b) obtaining a 4S-iota-carrageenan sulfatase from the culture medium obtained at step (a).

9. Production method according to claim 8, in which at step (a) when the bacterial population is a *Pseudoalteromonas carrageenovora bacterial population,* the culture medium contains lambda-carrageenan.

10. Production method according to claim 8, in which at step (a), when the bacterial population is a *Pseudoalteromonas haloplanktis bacterial population,* the culture medium contains iota-carrageenan and/or an iota-/nu-carrageenan mixture.

11. Production method according to claim 9, in which step (b) includes the following sub-steps:
(b1) centrifuging the culture medium obtained at step (a), and
(b2) obtaining 4S-iota-carrageenan sulfatase from the pellet obtained at step (bl).

12. Production method according to one of claims 8 to 11 which comprises, after step (b), the following additional step:
(c) determining the activity of the 4S-iota-carrageenan sulfatase.

13. Method for extracting a 4S-iota-carrageenan sulfatase from a population of marine red algae, comprising:
(i) isolating a bacterial population containing a 4S-iota-carrageenan sulfatase from the population of marine red algae, said bacterial population a *Pseudoalteromonas bacterial population,* advantageously a *Pseudoalteromonas haloplanktis* and/or a *Pseudoalteromonas carrageenovora bacterial population,* and
(ii) obtaining a 4S-iota-carrageenan sulfatase from the bacterial population obtained in step (i), according to the method defined in one of claims 8 to 12.

14. Method for converting a composition containing iota-carrageenan and/or hybrid iota-carrageenan into a composition which contains alpha-carrageenan and/or hybrid alpha-carrageenan, comprising the following steps:
(a) bringing together the composition containing iota-carrageenan and/or hybrid iota-carrageenan with a solution containing an isolated polypeptide comprising an amino acid sequence having at least 65% identity with the sequence SEQ ID N°2 or the sequence SEQ ID N°4 and having a 4S-iota-carrageenan sulfatase activity, under conditions suitable for the conversion of the composition, and
(b) optionally, recovering the composition containing the alpha-carrageenan and/or hybrid alpha-carrageenan obtained at step (a).

15. Conversion method according to claim 14, in which, at step (a), the solution containing the polypeptide is added to the reaction medium in order to increase the conversion rate.

16. Method according to claim 14 or 15, in which the amino acid sequence is selected from the group consisting of the sequence SEQ ID N°2 and the sequence SEQ ID N°4.

17. Method according to one of claims 14 to 16, in which the composition containing iota-carrageenan and/or hybrid iota-carrageenan is obtained from marine red algae.

18. Use of an isolated polypeptide comprising an amino acid sequence having at least 65% identity with the sequence SEQ ID N°2 or the sequence SEQ ID N°4, for the preparation of a texturizing agent comprising alpha-carrageenan and/or hybrid alpha-carrageenan.
